# EUROPEAN PATENT SPECIFICATION

(11) **EP 2 655 653 B1**
(45) Date of publication and mention of the grant of the patent: **18.11.2015**
(21) Application number: 11802971.9
(22) Date of filing: 08.12.2011
(51) Int. Cl.: C12Q 1/26

(54) **METHODS AND COMPOUNDS FOR DETECTING CANCER**
VERFAHREN UND VERBINDUNGEN ZUM NACHWEIS VON KREBS
PROCÉDÉS ET COMPOSÉS POUR DÉTECTER UN CANCER

(30) Priority: 20.12.2010 GB 201021494
(43) Date of publication of application: 30.10.2013
(73) Proprietor: University College Cardiff Consultants Limited, Cardiff, South Glamorgan CF24 0DE (GB)
(72) Inventor: WEEKS, Ian, Cardiff South Glamorgan CF23 6NE (GB); JAFFAR, Mohammed, Manchester Lancashire M20 4XU (GB); KNOX, Richard, Salisbury Wiltshire SP1 1HL (GB)
(74) Representative: Fyles, Julie Marie
(86) International application number: PCT/GB2011/052430
(87) International publication number: WO 2012/085532

(56) References cited:
- EP-A1- 1 445 602
- WO-A1-2004/009602
- WO-A1-2011/113018
- US-A1- 2007 185 188
- US-A1- 2009 012 031
- US-A1- 2010 047 839
- HUANG S T ET AL: "Synthesis of a new long-wavelength latent fluorimetric indicator for analytes determination in the DT-Diaphorase coupling dehydrogenase assay system", BIOSENSORS AND BIOELECTRONICS, ELSEVIER BV, NL, vol. 23, no. 12, 15 July 2008 (2008-07-15) , pages 1793-1798, XP022666043, ISSN: 0956-5663, DOI: 10.1016/J.BIOS.2008.02.015 [retrieved on 2008-03-04]
- BLANCHE E A ET AL: "Synthesis of potential prodrug systems for reductive activation. Prodrugs for anti-angiogenic isoflavones and VEGF receptor tyrosine kinase inhibitory oxindoles", TETRAHEDRON, ELSEVIER SCIENCE PUBLISHERS, AMSTERDAM, NL, vol. 65, no. 25, 20 June 2009 (2009-06-20) , pages 4894-4903, XP026129927, ISSN: 0040-4020, DOI: 10.1016/J.TET.2009.04.014 [retrieved on 2009-04-10]
- M. VOLPATO ET AL: "Chemical synthesis and biological evaluation of a NAD(P)H:quinone oxidoreductase-1 targeted tripartite quinone drug delivery system", MOLECULAR CANCER THERAPEUTICS, vol. 6, no. 12, 7 December 2007 (2007-12-07), pages 3122-3130, XP55029795, ISSN: 1535-7163, DOI: 10.1158/1535-7163.MCT-07-0519
- HERNICK MARCY ET AL: "Design, synthesis, and biological evaluation of indolequinone phosphoramidate prodrugs targeted to DT-diaphorase", JOURNAL OF MEDICINAL CHEMISTRY, AMERICAN CHEMICAL SOCIETY, US, vol. 45, no. 16, 1 August 2002 (2002-08-01), pages 3540-3548, XP002586859, ISSN: 0022-2623, DOI: 10.1021/JM020191B [retrieved on 2002-07-04]
- ELIZABETH SWANN ET AL: "Indolequinone antitumor agenty: Correlation between quinone structure and rate of metabolism by recombinant human NAD(P)H:Quinone oxidoreductase. Part 2", JOURNAL OF MEDICINAL CHEMISTRY, AMERICAN CHEMICAL SOCIETY, US, vol. 44, no. 20, 1 January 2001 (2001-01-01), pages 3311-3319, XP002530224, ISSN: 0022-2623, DOI: 10.1021/JM010884C [retrieved on 2001-08-31]
- SMITSKAMP-WILMS E ET AL: "Chemosensitivity to the indoloquinone EO9 is correlated with DT-diaphorase activity and its gene expression", BIOCHEMICAL PHARMACOLOGY, PERGAMON, OXFORD, GB, vol. 47, no. 8, 20 April 1994 (1994-04-20) , pages 1325-1332, XP025542846, ISSN: 0006-2952, DOI: 10.1016/0006-2952(94)90330-1 [retrieved on 1994-04-20]
- SANYAL SOMALI ET AL: "Polymorphisms in NQO1 and the clinical course of urinary bladder neoplasms", SCANDINAVIAN JOURNAL OF UROLOGY AND NEPHROLOGY, SCANDINAVIAN UNIVERSITY PRESS, STOCKHOLM, SE, vol. 41, no. 3, 1 January 2007 (2007-01-01), pages 182-190, XP009104832, ISSN: 0036-5599, DOI: 10.1080/00365590600991946

## Description

The present application relates to methods of diagnosing cancers, particularly bladder and prostate cancer, using compounds which are useful in the detection of NQO1or NQO2 expressing cells.

Bladder cancer is the ninth most common cancer worldwide. It is more prevalent in men than women. Worldwide an estimated 356,600 new cases of bladder cancer occur each year (2008), with approximately 20,000 deaths per year. The highest bladder cancer incidence rates are generally found in industrially developed countries, particularly in North America and Western Europe.

In developed countries approximately 90% of bladder cancers are transitional cell carcinomas (TCC, bladder warts) while the remaining 10% are squamous cell carcinomas and adenocarcinomas. Superficial transitional cell carcinoma tends to spread only within the bladder unless it is left untreated for a long period of time. TCC may spread along the lining of the bladder but does not penetrate deeply into the bladder (unless left untreated) and the cells are shed into the urine.

Superficial bladder tumours can be managed very effectively by repeated resection. The tumour is removed (resected) via a cystoscope which is passed up the urethra. This type of treatment is highly invasive. Superficial bladder tumours tend to recur intermittently and may require resection on a repeated basis. Invasive bladder cancer requires a more aggressive approach. In the early stages, the tumours can be resected surgically either by partial or complete removal of the bladder. This may require major surgery which will require the creation of an ileal conduit. Routine 'check cystoscopy' is used to detect the recurrence of tumours particularly in the early stages and further treatment initiated if required. The time interval between check cystoscopies is usually 3-4 months initially but may be increased if the bladder remains free of tumour at subsequent investigation. Check cystoscopy is recommended for a period of several years to ensure that the tumour has not returned. Approximately 85% of patients with bladder cancer suffer recurrence within 5 years, the majority of patients within 2 years. The high recurrence rate may, to a large extent, be attributed to the tumours in the bladder being present in multiple locations which may be missed on examination or are too small to be seen by the surgeon during initial resection. Hence there is a clinical need for a sensitive, specific and non-invasive means of bladder cancer detection.

The need for a diagnostic test to detect early stage bladder cancer (superficial transitional cell carcinoma) is warranted due to the high risk of recurrence (85%) following surgical or chemotherapeutic intervention in the first 5 years. In addition to methods requiring invasive examination and sampling, several non-invasive tests have been described. A recent review (Shariat et al, 2008) of such methods concluded that none of the tests reviewed met all of the criteria of an ideal tumour marker. Thus there is a need for a simple, rapid, accurate and non-invasive method for early detection of bladder cancer and the invention disclosed herein provides a solution to this problem.

Elevated levels of NAD(P)H:quinone reductase-1 (NQO1, E.C. 1.6.99.2) and other related redox enzymes in superficial bladder cancer have been detected when compared to invasive transitional cell carcinoma (Li et al, 2001, J. Urol., 166, 2500-2505; Choudry et al., 2001, Br. J. Cancer, 85, 1137-1146). This significant difference has been exploited to treat early stage bladder cancers by using NQO1-specific agents. Benzoquinone-drug conjugate systems have been developed to specifically target NQO1-rich tumour cells.

NQO1 is also over-expressed in the cells of various other types of cancer, including breast, non-small cell lung, pancreas, colon and prostate cancers, relative to normal cells.

The related enzyme NQO2 is also over-expressed in cancer cells, including those set out above.

US 2010/047839 and Biosensors and Bioelectronics, 23, 1793-1798 (2003), (Huang *et al*) relate to latent fluorimetric indicators for monitoring DT diaphorase (NQO1) activity. The compounds include 1,4-benzoquinone derivatives in which a fluorometric probe can be cleaved from the remainder of the molecule using DT diaphorase and NADH.

Molecular Cancer Therapeutics, 6(12), 3122-3130 (2007), Volpato *et al*, teaches that NQO1 is a target for therapeutic intervention because of its ability to convert certain quinones into cytotoxic species and that elevated levels of NQO1 have been found in several tumour types, for example non-small cell lung cancer and pancreatic cancers. The authors propose treating these cancers using compounds which are activated by NQO1 and which are reduced to release an inert metabolite and a therapeutic agent.

J. Med. Chem., 45, 3540-3548 (2002), Hernick *et al* relates to the design, synthesis and biological evaluation of prodrugs targeted to DT-diaphorase (DTD, NQO1).

J. Med. Chem., 44, 3311-3319 (2001), Swann *et al* teaches that NQO1 catalyses the reduction of quinones and is therefore able to protect cells against the toxic effects of quinones.and relates to a study of the reaction of series of indole quinones with NQO1.

Biochem. Pharmacol., 47(8), 1325-1332 (1994), Smitskamp-Wilms *et al* relates to a study of the compound EO9, one of a series of bioreductive indoloquinones, which is a substrate for human and rodent NQO (DTD) enzymes.

WO 2011/113018 relates to a method of measuring and modulating "biological time", which is defined as a mechanism by which a living system coordinates or synchronises biological processes. Biological time is said to be correlated with the concentration of redox-active molecules such as NCO1.

EP1445602 relates to methods and kits for the detection and determination of analytes using a redox reaction followed by fluorometric determination. The methods are carried out using a compound comprising a quenching group linked to a fluorophore.

Tetrahedron, 65(25), 4894-4903 (2009), Blanche *et al* relates to prodrug systems for use in cancer therapy. The systems disclosed include 1,4-benzoquinone derivatives which comprise a group D, where D is a drug residue and reduction of the compound leads to release of a drug DH. The document also discloses model systems in which the drug residue D is replaced by an oxochromene moiety.

US 2009/0012031 relates to compounds which target EZH2 expression in prostate cancer and WO 2004/009602 relates to pyrazolopyridine compounds which are useful as kinase inhibitors.

The present invention takes advantage of the over-expression of NQO1 and NQO2 in cancer cells relative to normal cells and relates to enzyme substrates that are activated by NQO1 or NQO2, resulting in a detectable signal in the presence of cancer cells. By contrast, minimal signal is observed in the absence of cancer cells (and thus greatly reduced amounts of NQO1 and/or NQO2).

### Statements of Invention

In a first aspect of the invention there is provided a method of determining the presence or absence, in a urine from a patient, of bladder or prostate cancer cells which over-express NQO1 and/or NQO2, the method comprising:
i. contacting the urine sample, or a processed derivative thereof, with a compound of general formula (I), (Ia), (Ib), (Ic), (Id), (Ie) or (If) or a salt of any thereof where applicable, wherein the biological sample contains or is suspected of containing bladder or prostate cancer cells which over-express NQO1 and/or NQO2;
ii. optionally, in the case where the bladder or prostate cancer cells over-express or are suspected of over-expressing NQO2, adding an NQO2 co-substrate to the sample; and
iii. determining the presence or absence of a compound of the formula:

   z-XH;

   or an ion of the formula:

   z-X⁻;

   wherein z and X are as defined in general formula (I), wherein presence of the compound or ion indicates the presence in the sample of cancer cells which over-express NQO1 and/or NQO2;
   wherein general formula (I) is: 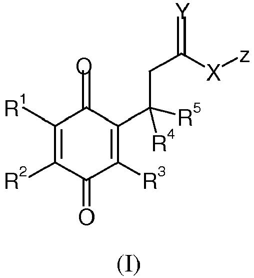
   wherein R¹, R², R³, R⁴ and R⁵ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
   R⁶ and R⁷ each independently represent hydrogen or C₁-C₆ alkyl optionally substituted with halo; or
   R¹ and R² together with the carbon atoms to which they are attached form a 5- or 6-membered optionally substituted aromatic, heteroaromatic, carbocyclic or heterocyclic ring system;
   X is O, S or NR⁸;
   R⁸ is hydrogen, or C₁-C₃ alkyl;
   Y is O, S or NR⁹;
   R⁹ is hydrogen, or C₁-C₃ alkyl;
   z is a moiety which is covalently linked to the remainder of the molecule and which, on reduction of the compound of general formula (I), is cleaved from the remainder of the molecule to form a detectable compound z-XH or ion z-X⁻;
   wherein the biological sample contains or is suspected of containing cancer cells which over-express NQO1 and/or NQO2;
   general formula (Ia) is: 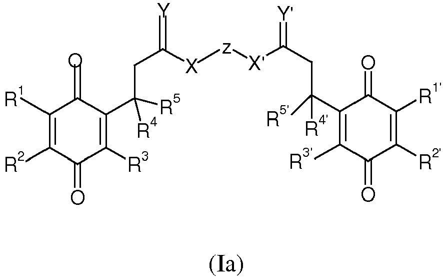
      wherein R¹, R², R³, R⁴, R⁵, X, Y and z are as defined for general formula (I);
      R^{1'}, R^{2'}, R^{3'}, R^{4'} and R^{5'} each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
      R⁶ and R⁷ each independently represent hydrogen or C₁-C₆ alkyl optionally substituted with halo; or
      R^{1'} and R^{2'} together with the carbon atoms to which they are attached form a 5- or 6-membered optionally substituted aromatic, heteroaromatic, carbocyclic or heterocyclic ring system;
      X' is O, S or NR⁸;
      R⁸ is hydrogen, or C₁-C₃ alkyl;
      Y' is O, S or NR⁹;
      R⁹ is hydrogen, or C₁-C₃ alkyl;
   general formula (Ib) is: 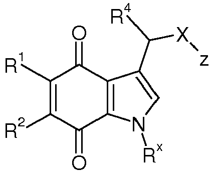 wherein R¹, R², R⁴, X and z are as defined for general formula (I) and R^{x} is H, or C₁-C₃ alkyl;
   general formula (Ic) is: 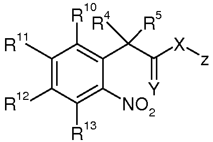
      wherein R⁴, R⁵, X, Y and z are as defined for general formula (I); and
      R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
      R⁶ and R⁷ each independently represent hydrogen or C₁-C₆ alkyl optionally substituted with halo;
   general formula (Id) is: 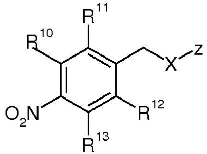
      wherein X and z are as defined for general formula (I); and
      R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
      R⁶ and R⁷ each independently represent hydrogen or C₁-C₆ alkyl optionally substituted with halo;
   general formula (Ie) is: 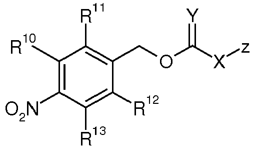
      wherein X, Y and z are as defined for general formula (I); and
      R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
      R⁶ and R⁷ each independently represent hydrogen or C₁-C₆ alkyl optionally substituted with halo;
   general formula (If) is: 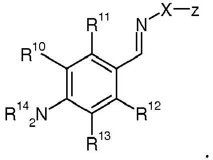
      wherein z is as defined for general formula (I);
      R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
      R⁶ and R⁷ each independently represent hydrogen or C₁-C₆ alkyl optionally substituted with halo;
      R¹⁴ is H or C₁-C₆ alkyl.

The compounds of general formulae (I), (Ia), (Ib), (Ic), (Id), (Ie) and (If) are susceptible to reduction to yield a product of formula z-XH or an anion of formula z-X⁻ and a reduced residue. As will be discussed in greater detail below, the moiety z is a detectable marker and the remainder of the molecule is chosen such that the detectable signal emitted by the group z is modified when it forms part of the compound of general formula (I), (Ia), (Ib), (Ic), (Id), (Ie) or (If) compared with the signal emitted by the compound z-XH or the ion z-X⁻.

Quinone or benzoquinone compounds of formula (I) and (Ia), indoles of formula (Ib), nitro-based compounds of general formula (Ic), (Id) and (Ie) and compounds of general formula (If) are all known in the art. For example, compounds of general formula (Ia) are taught by Huang et al, Org. Letters, 8(2), 2665-268 (2006) and compounds of general formulae (I), (Ib), (Ic), (Id) and (Ie) are discussed in Blanche et al, Tetrahedron, 65(25), 4892-4903 (2009).

The nature of the detectable moiety, i.e. whether it is a compound of the formula z-XH or an anion of formula z-X⁻ will depend upon the nature of the detectable marker z, the detection method used and the environment in which the detection method is conducted. Therefore, hereafter, references to a compound of formula z-XH should be taken also to comprehend an anion of formula z-X⁻. Thus, methods for the detection and/or quantification of a compound z-XH also include methods for the detection and/or quantification of an anion z-X⁻.

Further, reference to a processed derivative of a biological sample includes reference to a biological sample after it has been treated, typically for the purpose of preparing it for the method of the invention or preserving it prior to undertaking the said method and involves the use of conventional techniques well known to those skilled in the art of taking, preparing or preserving biological samples.

In the present specification "C₁-C₆ alkyl" refers to a straight or branched saturated hydrocarbon chain having one to six carbon atoms. Examples include methyl, ethyl, n-propyl, isopropyl, t-butyl and n-hexyl.

The term "C₁-C₃ alkyl" refers to an alkyl group having from 1 to 3 carbon atoms.

The term "aromatic" in the context of the present specification refers to a ring system with aromatic character having 5 or 6 ring carbon atoms. Aromatic groups may optionally be substituted with one or more substituents independently selected from halo, methyl, ethyl, methoxy, ethoxy, nitro and cyano. Phenyl is a particularly suitable aryl group.

The term "heteroaromatic" in the context of the specification refers to a ring system with aromatic character having 5 or 6 ring atoms, at least one of which is a heteroatom selected from N, O and S. Examples of heteroaromatic groups include pyridine, pyrimidine, furan, thiophene, oxazole, diazole and triazole. Heteroaromatic groups may optionally be substituted with one or more substituents independently selected from halo, methyl, ethyl, methoxy, ethoxy, nitro and cyano.

The term "carbocyclic" in the context of the present specification refers to a nonaromatic ring system having 5 or 6 ring carbon atoms. The ring may contain one or more carbon-carbon double bonds and the term therefore encompasses cycloalkyl and cycloalkenyl groups. Examples of carbocyclic groups include cyclohexyl, cyclopentyl and cyclohexenyl groups. Carbocyclic groups may optionally be substituted with one or more substituents independently selected from halo, methyl, ethyl, methoxy, ethoxy, nitro and cyano.

The term "heterocyclic" in the context of the present specification refers to a nonaromatic ring system having 5 or 6 ring atoms, at least one of which is a heteroatom seelceted form N, O and S. The ring may contain one or more double bonds. Examples of heterocyclic groups include piperidinyl, piperazinyl, morpholinyl and tetrahydrofuryl groups. heterocyclic groups may optionally be substituted with one or more substituents independently selected from halo, methyl, ethyl, methoxy, ethoxy, nitro and cyano.

The term "halo" refers to fluoro, chloro, bromo or iodo.

The term "reactive substituents" as used herein refers to a substituent which is capable of reacting with a pendant group of a solid substrate such as a membrane, nanoparticle or polymer surface or on a protein or polypeptide. There are many reactions by which compounds of formula (I) can be linked to solid substrates, and the reactive substituents will, of course, depend upon the nature of the pendant group and the chosen reaction. Suitable reactive substituents inlcude halo, hydroxy, thiol, amino, carbonyl, carboxyl, cyano, azido, C₂-C₆ alkenyl and C₂-C₆ alkynyl groups, with particularly suitable reactive substituents being halo, hydroxyl, thiol, amino, carbonyl and carboxy.

The biological sample may be a biopsy sample, or a processed derivative thereof, taken from a patient who has or is suspected of having breast, non-small cell lung, pancreas, colon, cervical, testicular, prostate or bladder cancer. Alternatively, the biological sample may be a processed derivative of the biopsy sample, for example cells harvested from a biopsy sample, for example by centrifugation, and if necessary re-suspended in an alternative medium.

The method is particularly suitable for diagnosing prostate or bladder cancer, especially superficial bladder tumours, since cells are shed into the urine which can thus be used as the biological sample. Alternatively, a processed derivative of a urine sample may be used as the biological sample. An example of such a processed derivative is cells harvested from a urine sample and, if necessary re-suspended in an alternative medium.

In a preferred method of the invention the number of cells in said sample, or a test amount thereof, is also determined such that the NQO1/NQO2 activity can be expressed per cell. Said cells may be present in the crude sample or may be enriched, isolated or purified from the crude sample. This may be achieved by centrifugation, filtration or other methods disclosed in the scientific literature. Ideally, enrichment or purification is undertaken using a ligand binding method, such as, but without limitation, the use of paramagnetic particles coated with an antibody, receptor or other binding partner for a selected cell surface marker of interest.

In a preferred method of the invention the presence or absence of z-XH or z-X⁻ is determined as an amount represented by a ratio of the z-XH or z-X⁻ concentration of the sample to that of a negative assay control containing either no cells, no NQO1 and/or NQO2 expressing cells or normal cells which may because of their nature express very low levels of the enzymes. Ideally, different amounts of this ratio are correlated with known cancer cell staging techniques such that a simple *in vitro* assay can be used to reliably inform a clinician about, not only the existence of a cancer, but also its likely progression. In a further preferred method of the invention, greater NQO1 activity is seen in those samples from patients with later stage tumours.

In a further preferred method of the invention the assay protocol involves centrifuging a sample containing cells thought to be over-expressing NQO1 and/or NQO2, removing the supernatant, re-suspending the cells in selected buffer, incubating with a compound of general formula (I), and assaying for z-XH or z-X⁻ as above. Ideally the incubation is undertaken for approximately 3min. It follows that the assay of the invention can be undertaken relatively straightforwardly and takes only a short period of time, favouring a point-of-care application.

As described in greater detail below, the marker z may be a chromophore or a luminophore (e.g. a fluorescent, phosphorescent, bioluminescent or chemiluminescent marker); or a modulator of emissions from a fluorescent, phosphorescent, chemiluminescent or bioluminescent molecule or ion; or a co-factor for a chemiluminescent or bioluminescent reaction. Alternatively, the marker may be a detectable micro or nanoparticle such as, but without limitation, a coloured or magnetic particle. The method by which the presence or absence of the compound z-XH or z-X⁻ is determined will vary depending upon the nature of the detectable marker z. For example, changes in fluorescence intensity or wavelength can be monitored using a fluorimeter and analogous changes in chemiluminescence monitored using a luminometer. If required, the product of the enzyme reaction may be isolated, for example by liquid chromatography, prior to detection and/or quantitation.

The cleaved compound z-XH or z-X⁻ may alternatively be detectable by its ability to bind to a capture moiety and the z-XH or z-X⁻ and capture moiety binding pairs may comprise, for example, avidin or streptavidin and biotin or an antibody/antigen binding pair such as fluorescein/anti-fluoresecein.

As also described in more detail below, the cleaved compound z-XH or z-X⁻ may alternatively be detectable by its ability to bind to a capture moiety and the z-XH or z-X⁻ and capture moiety binding pairs may comprise, for example, avidin or streptavidin and biotin or an antibody/antigen binding pair such as fluorescein/anti-fluorescein.

The compounds of general formula (I), (Ia), (Ib), (Ic), (Id), (Ie) and (If) are substrates for NQO1 and NQO2 and are reduced by these enzymes. Reduction results in cleavage of the marker moiety z which can then be detected. Cleavage occurs according to Scheme 1 below, which illustrates the reaction mechanism for compounds of general formula (I). Reduction of compounds of general formulae (Ia), (Ib), (Ic), (Id), (Ie) and (If) proceeds in a similar manner.

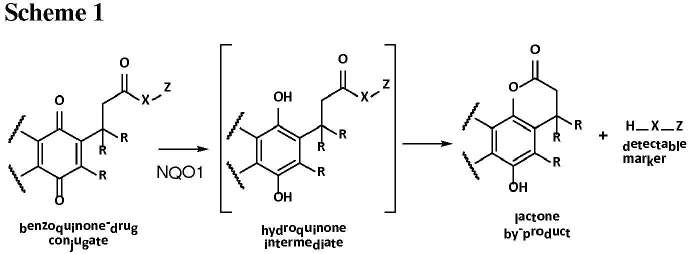

The compounds of the invention are particularly useful in the detection of cancer, since the quinone moiety is a substrate for the enzymes NQO1 and NQO2, which are over-expressed in cancer cells. The compounds are particularly useful for detecting prostate and superficial bladder cancer because such cells are shed into the urine and a diagnostic test can therefore be carried out on a urine sample or on a processed derivative of a urine sample such as cells harvested from the sample, without the need for an invasive procedure. In the case where the cancer cells over-express NQO2, it may be necessary to add to the sample or processed derivative an NQO2 co-substrate such as *N*-ribosyldihydronicotinamide (NRH) or 1-methyl1-3-carboxamidopyridinium iodide especially when reduced to the 1,4-dihydroptridine derivative or 1-carbamoylmethyl-3-carbamoyl-1,4-dihydropyridine, all of which act as a co-substrate for NQO2. Other NQO2 co-substrates are available and known to those skilled in the art such as those described in Knox et al cancer res. 60 pp 4179-4186, 2000. It is not usually necessary to add a co-substrate in cases where the over-expression of NQO1 is to be detected since NAD(P)H, the co-substrate for NQO1, is present in all cells. However, the latter co-substrate, or its equivalent, can be used in situations where the NQO1 has previously been isolated from the biological sample prior to measurement of activity, or the cells to be investigated have been lysed.

The discussion below relates to the detection of compounds z-XH or ions z-X⁻. In this discussion, references to a compound of formula (I) apply also to compounds of general formulae (Ia), (Ib), (Ic), (Id), (Ie) and (If).

Suitable detectable compounds z-XH or ions z-X⁻ include, in particular, chromophores and luminophores, for example fluorescent, phosphorescent, chemiluminescent or bioluminescent molecules or ions; or modulators of emissions from fluorescent, phosphorescent, chemiluminescent or bioluminescent molecules or ions; or a co-factor for a chemiluminescent or bioluminescent reaction. In particular, the moiety z may be chosen such that its optical properties change when it is cleaved from the remainder of the compound of general formula (I) to form the compound z-XH or the ion z-X⁻.

The change in optical properties may be, for example, a detectable change in the wavelength of emitted light, the removal of a quenching effect exerted by quinone moiety of general formula (I) or, in the case of co-factors, a modulation of their activity.

Most commonly, when the moiety z is a luminophore or chromophore, the change in its optical properties on cleavage from the remainder of the compound of formula (I) is based on two underlying mechanisms. The first is due to the 'electron-withdrawing' attachment (via the C-O-X link) of the active signalling moiety and the other is due to quenching achieved via the (pseudo) π-stacking phenomenon. These mechanisms are illustrated in Schemes 2A and 2B, using coumarin as an example.

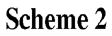

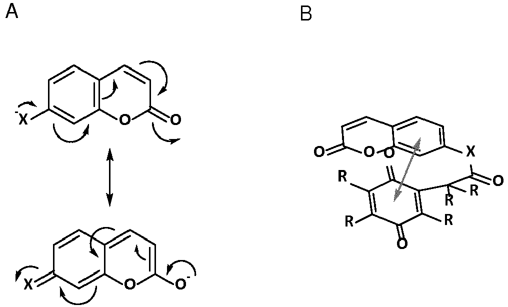

In Scheme 2A, the coumarin anion undergoes resonance tautomerism resulting in fluorescence output. If the group X is attached to an electron withdrawing quinone moiety as in general formula (I), the fluorescence is switched off.

Scheme 2B shows pseudo π-stacking fluorescent quenching between the quinone and the aromatic coumarin moieties.

In an alternative embodiment, the moiety z may comprise a detectable label, for example a detectable particle especially a detectable micro- or nano-particle such as, but without limitation, a coloured latex microparticle, gold nanoparticle or magnetic particle, as well as numerous detectable molecules, all of which are well known to those of skill in the art. The method by which the presence or absence of the compound z-XH or ion z-X⁻ is determined will vary depending upon the nature of the detectable moiety z. For example, changes in fluorescence intensity or wavelength can be monitored using a fluorimeter and analogous changes in chemiluminescence monitored using a luminometer. If required, the product of the enzyme reaction may be isolated, for example by liquid chromatography, prior to detection and/or quantitation.

Alternatively, the cleaved compound z-XH or ion z-X⁻ may be detectable by its ability to bind to a capture moiety. In this case, the moiety z may simply comprise a binding portion which selectively binds the capture moiety and the capture moiety may comprise a binding partner for the moiety z and a detectable label, for example a detectable particle as described above.

When z comprises a detectable label as described above, it may further comprise a binding portion which selectively binds a capture moiety.

Examples of suitable binding pairs which may be used in this type of embodiment are known, for example biotin and either avidin or streptavidin and antigen/antibody binding pairs.

Thus, in some cases, one of z and the capture moiety may comprise biotin or a biotin derivative and the other may comprise avidin or streptavidin or a derivative thereof. Alternatively, one of z and the capture moiety may comprise an antigen and the other an antibody specific for the antigen, for example, fluorescein/anti-fluorescein. Other examples of suitable binding pairs are well known in the art.

One way of detecting such a marker is to immobilize the complex formed by the marker z and the capture moiety on a solid substrate and to detect the label on the said solid substrate. Thus, in one embodiment, the capture moiety will be immobilized on a solid substrate such as beads, fibres or a membrane and the moiety z will comprise a detectable label.

If NQO1 is present in the sample, the moiety z will be cleaved from the compound of general formula (I) and the free z-XH or z-X⁻ will bind to the immobilized capture moiety allowing detection of the label.

Alternatively, a binding assay format may be used and this will be particularly suitable when moiety z simply comprises a binding partner for the capture moiety. In this case an appropriate labeled secondary binding reagent is used to monitor the occupancy of the capture moiety.

In some cases, the compound of formula (I) may be immobilized on a solid substrate at a first location, for example by covalent attachment involving any of the groups R¹ to R⁵ or suitable derivatives thereof, and a capture molecule immobilized at a second location. Suitable derivatives and methods for generally immobilising molecules to solid supports are well-known to those skilled in the art. If NQO1 or NQO2 is present in the sample, the compound of formula (I) will be reduced, the moiety z will be cleaved from the residue of the compound of general formula (I) and will be free to move to the second location where it can be captured and detected.

The detection methods described above may be qualitative or quantitiative. The quantitative detection of the compound z-XH or z-X⁻ makes it possible to determine the severity of the cancer and to monitor the effectiveness of any treatment.

In a further aspect of the invention there is provided a kit for diagnosing a cancer which over-expresses NQO1 and/or NQO2, the kit comprising a composition comprising a compound of general formula (I), (Ia), (Ib), (Ic), (Id), (Ie) or (If) in a suitable container; instructions for using the kit and optionally a composition comprising an NQO2 co-substrate in a suitable container.

Specific examples of marker moieties z include fluorescein, 2-oxo-2H-1-benzopyranyl and 4-methyl-2-oxo-2H-chromen-7-yl.

In a particularly suitable embodiment, the biological sample is contacted with a compound of general formula (I).

In suitable compounds of general formula (I) independently or in any combination: R¹, R², R³, R⁴ and R⁵ are each independently hydrogen, methyl or ethyl;
X is O or NH; and
YisO.

In more suitable compounds R¹, R², R³, R⁴ and R⁵ are each independently hydrogen or methyl and still more suitably:
R¹ and R² are both methyl;
R³ is hydrogen or methyl; and
R⁴ and R⁵ are the same and may be either hydrogen or methyl.

Particularly suitable compounds of general formula (I) include:
2-Oxo-2H-1-benzopyran-7-yl 3-methyl-3-(2,4,5-trimethyl-3,6-dioxo-cyclohexa-1,4-dienyl)-butanoate (R¹=R²=R³=R⁴=R⁵=Me);
2-Oxo-2H-1-benzopyran-7-yl 3-(4,5-dimethyl-3,6-dioxo-cyclohexa-1,4-dienyl)-3-methyl-butanoate (R¹=R²=R⁴=R⁵=Me; R³=H);
2-Oxo-2H-1-benzopyran-7-yl 3-(2,4,5-trimethyl-3,6-dioxo-cyclohexa-1,4-dienyl)-propanoate (R¹=R²=R³=Me; R⁴=R⁵=H); and
4-Methyl-2-oxo-2H-chromen-7-yl 3-methyl-3-(2,4,5-trimethyl-3,6-dioxocyclohexa-1,4-dienyl)butanoate (R¹= R²=R³=R⁴=R⁵=Me).

In separate alternative embodiments, the sample is contacted with a compound of general formula (Ia), a compound of general formula (Ib), a compound of general formula (Ic), a compound of general formula (Id), a compound of general formula (Ie) or a compound of general formula (If).

In the compounds of general formula (Ia), particularly suitable values for R¹, R², R³, R⁴, R⁵, X, Y and z are as defined for general formula (I), while particularly suitable values for R^{1'}, R^{2'}, R^{3'}, R^{4'} and R^{5'}, X' and Y' are as defined for R¹, R², R³, R⁴, R⁵, X and Y of general formula (I).

In compounds of general formula (Ib), particularly suitable values of R¹, R², R³, R⁴, R⁵, X, Y and z are as defined for general formula (I).

In compounds of general formula (Ic), particularly suitable values of R⁴, R⁵, X, Y and z are as defined for general formula (I);

In compounds of general formula (Id), particularly suitable values of X and z are as defined for general formula (I).

In compounds of general formula (Ie), particularly suitable values of X, Y and z are as defined for general formula (I);

In compounds of general formula (If), particularly suitable values of X and z are as defined for general formula (I) and R¹⁰ is suitably H or methyl.

In more suitable compounds of general formulae (Ic), (Id), (Ie) and (If), each of R¹⁰, R¹¹, R¹² and R¹³ independently represents H or C₁-C₆ alkyl, particularly H or methyl and most suitably H.

Compounds of general formula (I) may be prepared from compounds of general formula (II): 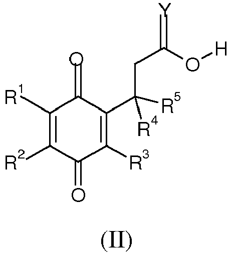 wherein R¹, R², R³, R⁴, R⁵ and Y are as defined for general formula (I); by reaction with a compound of formula (III):

z-XH or z-X⁻ (III)

where X and z are as defined above for general formula (I).

Typically, this reaction is carried out in the presence of a coupling reagent such as dicyclohexylcarbodiimide (DCC) and a base such as 4-dimethylaminopyridine (DMAP). The reaction may be conducted at a temperature of about 15-30°C, typically at room temperature. When X is NR⁸, the reaction may be carried out in the presence of DCC and N-hydroxysuccinimide and proceeds in a similar manner to a conventional peptide coupling reaction.

Compounds of general formula (II) may be prepared from compounds of general formula (IV): 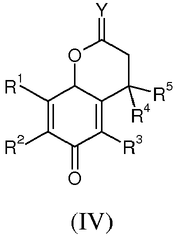 wherein R¹, R², ^{R}3, R⁴, R⁵, and Y are as defined for general formula (I);
by reaction with N-bromosuccinimide in acetonitrile, followed by the addition of water. The reaction may be conducted at a temperature of about 15-30°C, typically at room temperature.

Compounds of general formula (IV) may be prepared by reacting a compound of general formula (VI): 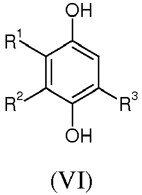 wherein R¹, R² and R³ are as defined for general formula (I);
with a compound of general formula (VII): 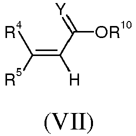 wherein Y, R⁴ and R⁵ are as defined for general formula (I).

The reaction may be conducted under acidic conditions, for example in the presence of methane sulfonic acid and at a temperature of from about 60-100°C, more usually 70-90°C.

Compounds of general formulae (VI) and (VII) are readily available or can be prepared by methods well known to those of skill in the art.

As already mentioned above, compounds of general formulae (Ia) are taught by Huang et al, Org. Letters, 8(2), 2665-268 (2006) and compounds of general formulae (Ib), (Ic), (Id) and (Ie) are discussed in Blanche et al, Tetrahedron, 65(25), 4892-4903 (2009). Methods for the preparation of these compounds are taught in these references directly or are described in references cited in those documents.

The compounds of general formula (I) and of general formulae (Ia), (Ib), (Ic), (Id), (Ie) and (If) are of use in methods for diagnosing cancer.

Examples of cancers which the compounds of general formula (I) and general formulae (Ia), (Ib), (Ic), (Id), (Ie) and (If) may be used to diagnose include prostrate cancers and urological malignancies where tumour cells may be present in urine. Therefore, suitably, the cancer is prostate cancer or more suitably, bladder cancer, especially superficial bladder tumours.

The Invention will now be described in greater detail with reference to the Examples below and to the drawings in which:

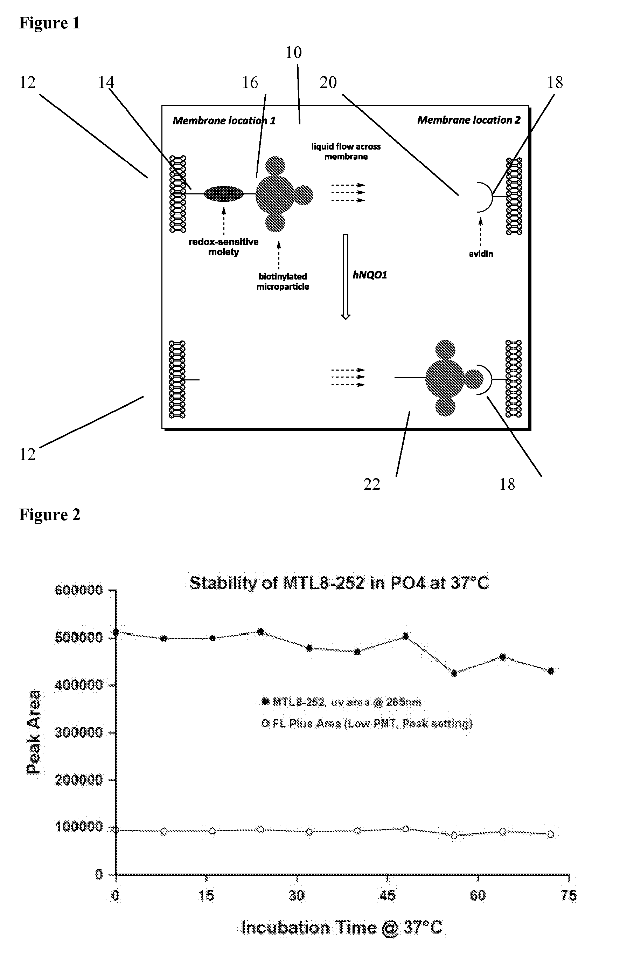

FIGURE 1 shows an example of an assay format in which NQO1 activity can be detected or quantified by its ability to cleave an immobilised, detectable particle from a solid-phase attachment by measurement of particles at the initial location, final location or both
FIGURE 2 is a plot showing the UV absorbance (at 265nm) (●) and fluormetric signal (λₑₓ410nm; λₑₘ550nm) (○) of **MTL8-252** (0.1M) in the presence of phosphate buffer (10mM) at 37°C monitored over a period of 75 mins as detected by the UPLC spectrophotometer. The plot demonstrates that **MTL8-252** is significantly stable in phosphate buffer for at least 1 hour.

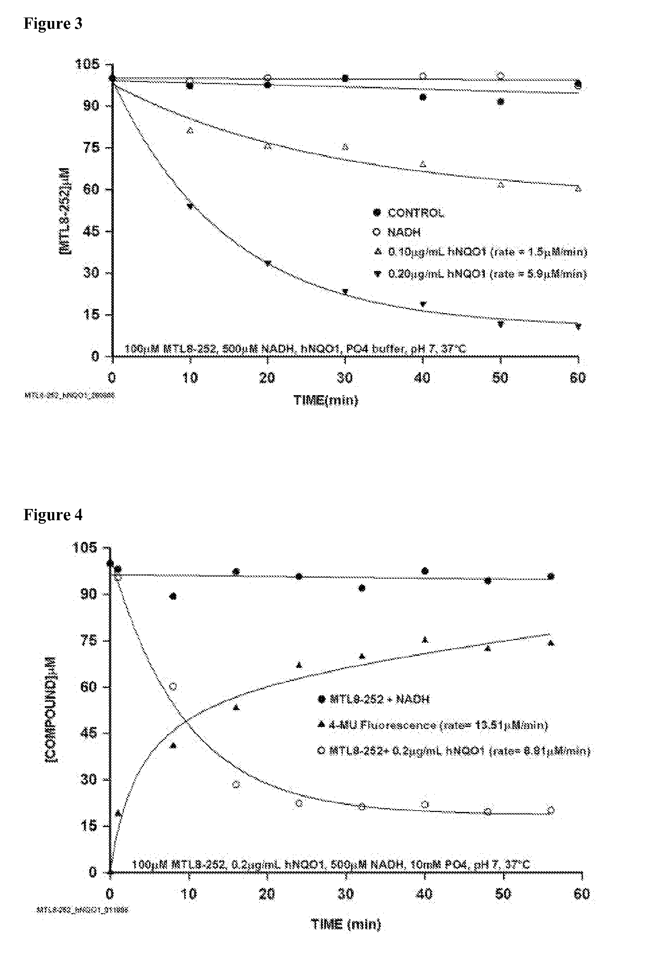

FIGURE 3 is a UV plot showing the disappearance of MTL8-252 (100µM) monitored at an absorbance of 265nm at 265nm at pH7 and at 37°C over time. Control (●) denotes the substrate in phosphate buffer alone; (○) denotes the substrate in buffer and NADH (500µM); (Δ) denotes the substrate in the presence of hNQO1 (0.10µg/mL) and NADH (500µM) and (▼) denotes the substrate in the presence of hNQO1 (0.20µg/mL) and NADH (500µM). The plot indicates that MTL8-252 is an excellent substrate for hNQO1.
FIGURE 4 is a plot as showing the disappearance of MTL8-252 and the appearance of 4MU in the presence and absence of hNQO1 over time using a UPLC assay. (○) represents the disappearance of MTL8-252 (initial conc 100µM) in the presence of hNQO1 (0.2µg/mL) and NADH (500µM); (▲) represents the appearance of 4MU as detected by its fluorescence; (●) is the control experiment in which MTL8-252 was incubated with NADH. The rate of disappearance of Compound was calculated at 13.51µM/min and the rate of appearance of 4MU was calculated at 8.41µM/min.

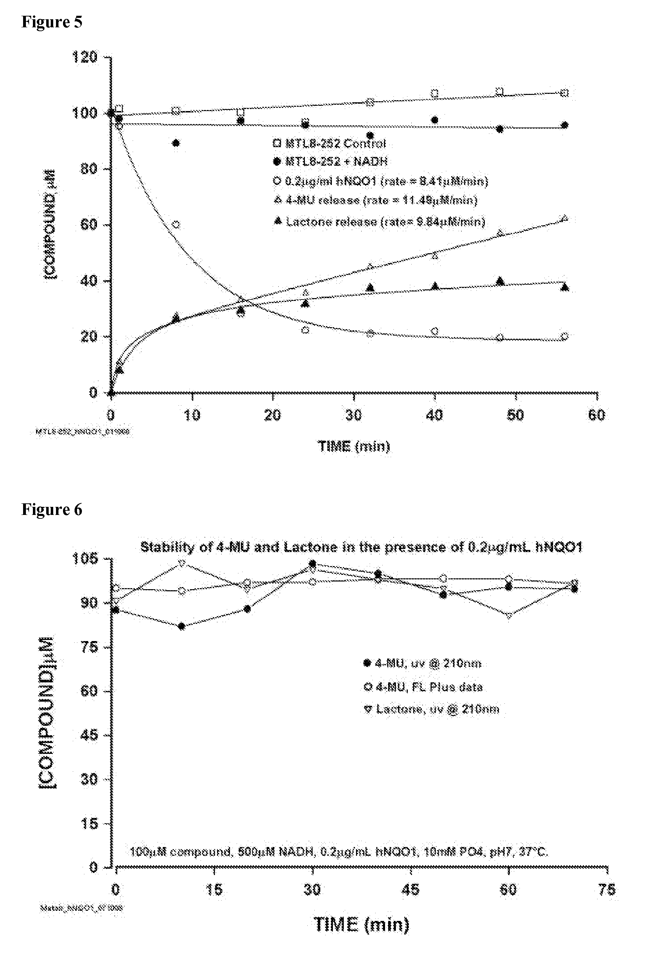

FIGURE 5 is a re-plot of the data shown in figure 6 but in which the concentrations were measured using UV (at 210nm) rather than the fluorescence over time. (□) represents MTL8-252 alone; (●) represents MTL8-252 in the presence of NADH; (○) represents the disappearance of MTL8-252 in the presence of hNQO1 and NADH; (Δ) represents the appearance of 4MU (release of 4MU from the activation process - MTL8-252+NADH+hNQO1); (▲) represents the appearance of the lactone byproduct (formation of the lactone from the activation process). The rate of disappearance of MTL8-252 was calculated at 8.41µg/min. The rate of appearance of 4MU was calculated at 11.48µg/min and the rate of appearance of the lactone was measured at 9.84µg/min.
FIGURE 6 is a stability plot, over time, when 4MU (100µM) and the lactone (100µM) were incubated with hNQO1 (0.2mg/µL) and NADH (500mM). (●) represents the 4MU as measured by UV at 210nm; (○) represents the 4MU as measured by fluorescence (FL Plus data); (∇) represents the lactone as measured by UV at 210nm. The data indicates that both 4MU and the lactone are stable in the presence of hNQO1 (i.e., not affected/activated by hNQO1).

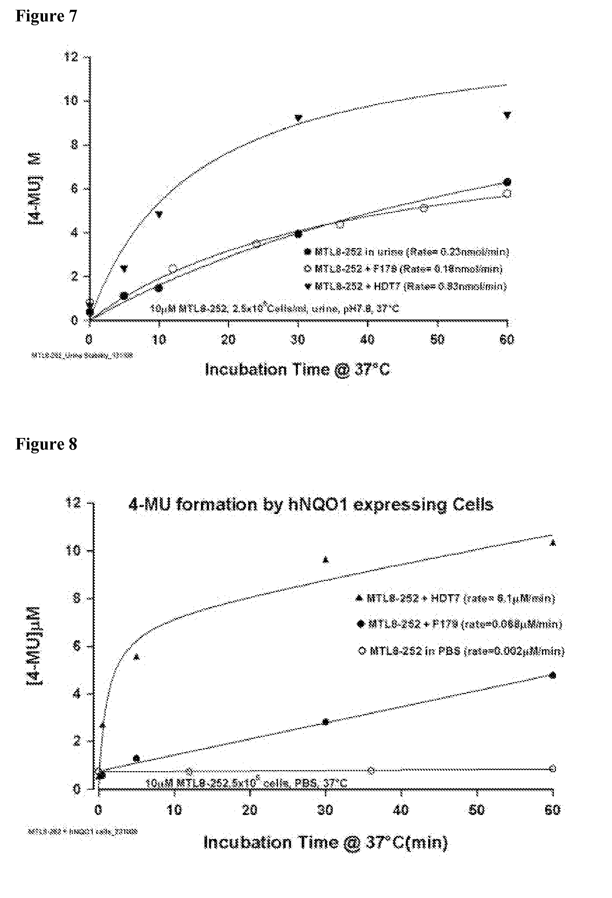

FIGURE 7 is a luminescence spectrophotometric plot showing the rates of formation of 4MU with time when MTL8-252 ()100mM) is incubated with hNQO1-expressing (hDT7; 2.5x10⁵ cells/Ml) and non-expressing hNQO1 cell lines (F170; 2.5x10⁵ cells/mL). (●) represents the Compound in urine alone: (▼) represents the compound with hDT7 NQO1-expressing cell line: (○) represents MTL8-252 with F179 - non/null expressing NQO1 cell lines. The rate of formation of 4MU in the urine alone and in the F179 cells is 0.32nmol/min and 0.18nmol/mL respectively. The rate of formation of 4MU in the hDT7 cells is 0.83nmol/mL.

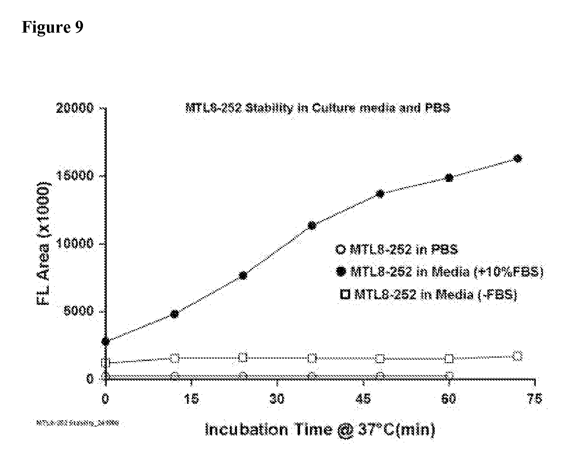

FIGURE 8 is a spectrophotometric plot of a repeat of figure 9 using an FL-Plus detector setting (λex=360nm; λem=450nm) - detecting the fluorescence of the released 4MU. The control experiment is represented by (○) which is MTL8-252 (10µM) in PBS alone; (▲) represents MTL8-252 (10µM) in PBS incubated with hDT7 NQO1-expressing cell line (5x10⁵ cells/mL); (●) represents MTL8-252 (10µM) in PBS incubated with the null expressing cell line F179 (5x10⁵cells/mL). The initial rate of release of 4MU in the hDT7 and F179 cells was calculated at 6.1µM/min and 0.068µM/min respectively. The rate of release of 4MU from the control experiment was 0.002µM/min.
FIGURE 9 is a spectrophotometric plot showing the stability of MTL8-252 in PBS (○); in media with 10% foetal bovine serum (FBS) (●) and in media without FBS (□). MTL8-252 is very stable in PBS and in media without FBS.

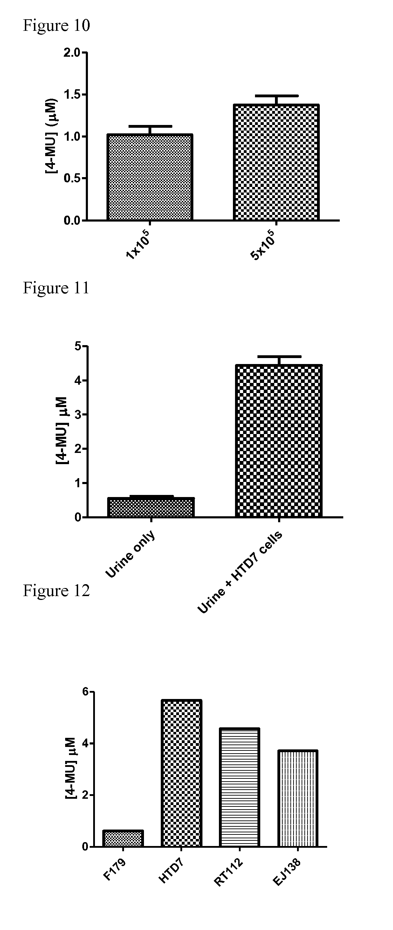

FIGURES 10 and 11 are plots showing the precision of an example assay. The assay was performed on replicate samples of NQO1-producing HDT7 cells spiked into either culture medium (DMEM) (figure 10) or urine (figure 11). Figure 10 shows the precision of triplicate determinations of 1 x 10⁵ or 5 x 10⁵ cells indicated spiked into culture medium. Figure 11 shows the precision of duplicate measurements of HDT7 10⁶ cells spiked into urine. Error bars are +1 SEM and the experiments demonstrate that the cells can be measured in either culture medium or urine without any adverse effect on precision.

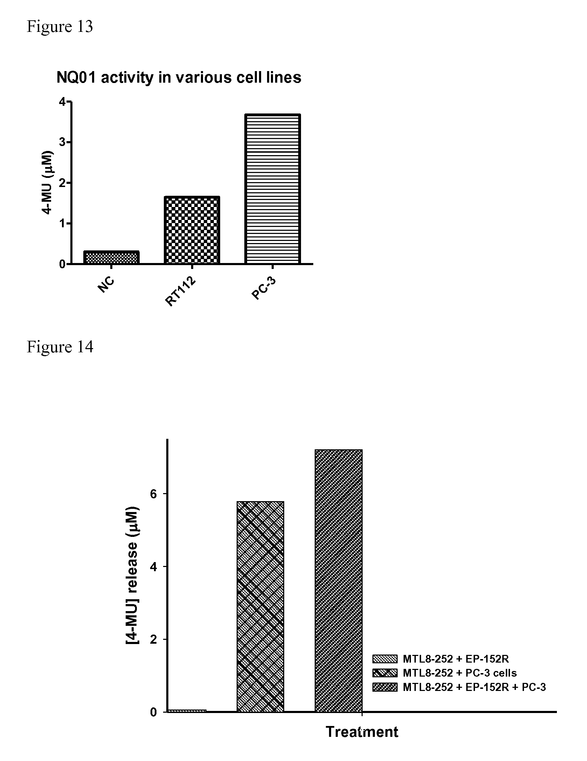

FIGURES 12 and 13 are plots showing the example assay could be used to assay a number of different types of cells for NQO1 activity, namely HDT7 and F179 (NQO1-producing and null engineered cell lines, respectively), human bladder cancer cell lines EJ138 and RT112 and human prostate cancer cell line PC3. Similar numbers of cells were used within each experiment to permit comparison. NC represents a non-cellular negative control.
FIGURE 14 shows the example assay can discriminate between NQO1 and NQO2 activity. Prostate carcinoma cells (as exemplified by PC3 cells) express both NQO1 and NQO2 activity. However, the latter is only activated by the presence of a co-substrate not naturally present in the cells (EP-0152R, 1-carbamoylmethyl-3-carbamoyl-1,4-dihydronicotinamide). It is known that in the absence of EP-152R hNQO2 is inactive. Figure 14 shows that there is a 1.2-fold increase in the formation of 4-MU by addition of a hNQO2 selective co-substrate. This additional 4-MU release indicates that the assay, when supplemented with hNQO2 co-substrate, can be used to detect tumour cells that, additionally or alternatively, express the hNQO2 enzyme. In the former instance, the supplemented assay can be used to provide a stronger assay signal and in the latter instance to detect hNQO2 expressing cancers only.

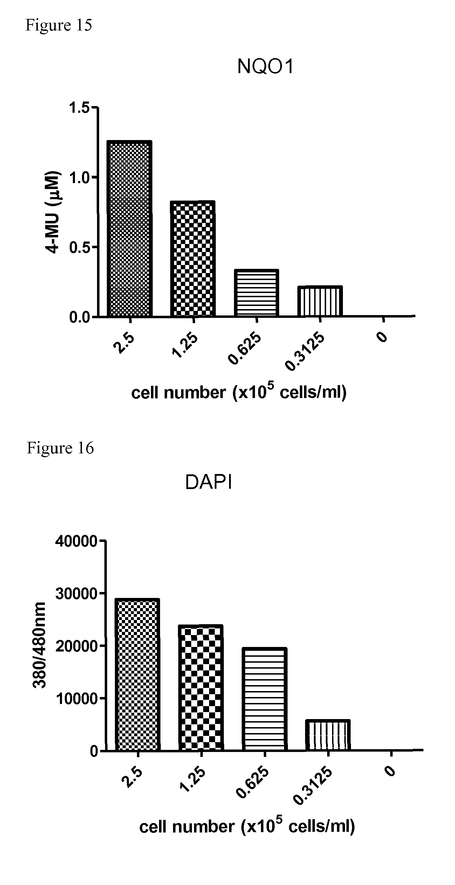

FIGUREs 15 and 16 shows the quantitative nature of the example assay. 2.5 x 10⁵, 1.25 x 10⁵, 0.625 x 10⁵, 0.3125 x 10⁵ RT112 cells were assayed (figure 15). Contemporaneously, the cells were also exposed to 4',6-diamidino-2-phenylindole (DAPI), using established protocols, to permit quantitation of total cell number by measurement of cellular DAPI fluorescence intensity in the same fluorimeter (figure 16). Thus, assessment of the data in figures 15 and 16 enables one to determine the amount of signal per cell and the number of cells in a sample producing said signal. This is particularly advantageous where the NQO1 or NQO2 activity is desired to be determined on a "per cell" basis.

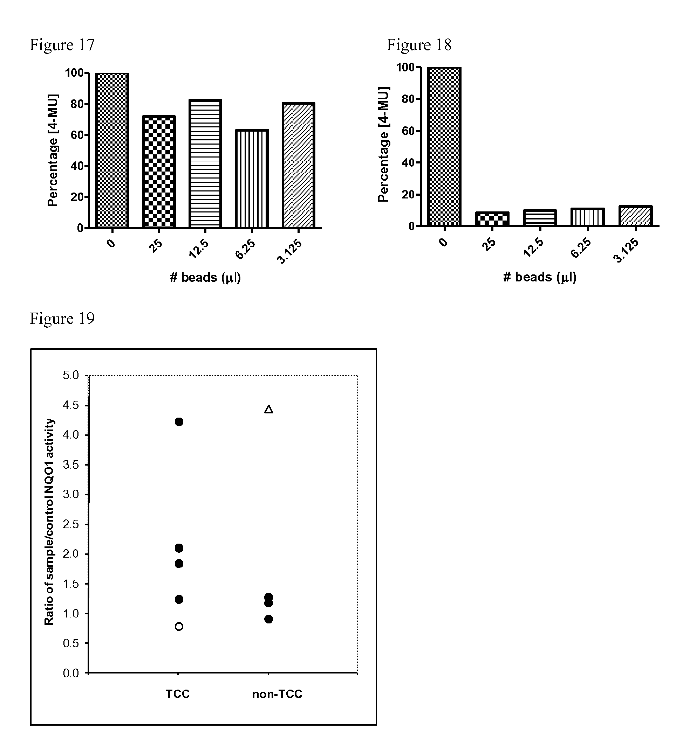

FIGURES 17 and 18 show the results of a modified version of the assay where sensitivity and/or specificity of the assay is improved by enrichment/isolation/purification of cells prior to assaying. Figure 17 shows the compatibility of the present invention with such procedures. Here, samples containing cancer cells carrying the Ber-EP4 epithelial antigen were isolated using magnetisable (paramagnetic) particles coated with antibodies to this antigen (Invitrogen, Dynal AS, Oslo, Norway). A suspension of particle/cell complexes was obtained which was processed in the example assay protocol reported in figures 10-16. Figure 17 shows that recovery of NQO1 activity in RT112 bladder cancer cells is approximately 60 - 70% relative to the activity of the total number of cells present (100 %, represented by the 0 beads bar in the figure) over a range of particle densities (illustrated by the volume of Manufacturer's bead stock solution (4 x 10⁶ particles/ml) added). Figure 18 shows the comparable results for HDT7 cells which express NQO1 but do not express human Ber-EP4 antigen. The data illustrates the ability to specifically enrich the epithelial cell population.
FIGURE 19 shows the results obtained when the assay was used on samples obtained from a clinical environment. Results are expressed as the ratio of 4-MU concentration of the samples to that of a negative assay control and correlated with the eventual clinical diagnosis (transitional cell carcinoma or not). The open triangle represents a sample containing significant amounts of debris suggesting that the use of immunoextraction as described herein with reference to figure 17 would be beneficial for increasing specificity. The open circle represents a sample from a patient subsequently diagnosed with very early stage Ta bladder cancer and it is likely that there were insufficient cells for reliable analysis of this sample.
TABLE 2 shows the assay results for patients already diagnosed with bladder cancer but as yet untreated. Results are expressed as the ratio of 4-MU concentration of the samples to that of a negative assay control. Sample 89 in this series could not be assayed due to the presence of gross haematuria.
TABLE 3 shows the NQOlassay results for patients diagnosed with prostate carcinoma. These samples were collected following digital rectal examination.

One type of assay format is illustrated in Figure 1. In the upper half of the figure (12) represents a first defined location on a suitable membrane (10) to which a detectable biotinylated microparticle (16; a "z" moiety) is attached via a NQO1 active substrate moiety (14, "redox-sensitive moiety"). Thus (14) and (16) together comprise a compound of Formula (I). In the absence of human NQO1 (hNQO1) the detectable particle (16) remains immobilised at the first defined location (12) when a fluid flow is induced across the membrane in the direction of a second defined location (18). Thus there is no capture of the biotinylated microparticles by the avidin (20) immobilised at the second location (18). By contrast the lower half of the figure illustrates the effect of prior cleavage of the redox-sensitive moiety (14) by the action of hNQO1 in a sample applied to the first location (12). In this case, subsequent induction of fluid flow across the membrane results in migration of the detectable, biotinylated microparticles (16) with subsequent capture by the avidin moieties (20) immobilised at the second location (18). Detection and/or quantitation of microparticles at the first (12) or second (18) defined locations relative to the other respective location is thus an indicator of the presence or absence, or quantity of, hNQO1 in the sample applied to the first location (12) prior to the induction of fluid flow.

The invention will now be described in greater detail with reference to the examples.

### General scheme for the synthesis of Compounds of general formula (I)

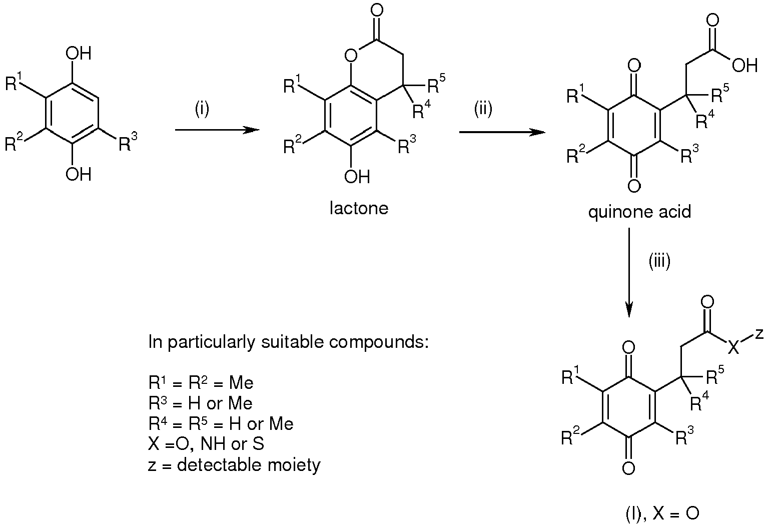

*Reagents and conditions:* (i) appropriate acrylate, MeSO₃H, 70-90°C, 2-5h; (ii) NBS, MeCN:H₂O, 1.5-3h, RT; (iii) DCC, DMAP, Z-XH, DCM, 16-24h, RT

### Experimental

Chemicals and reagents were obtained from Aldrich Chemical Co., Dorset UK, Lancaster Synthesis Ltd, Lancashire, UK and VWR International, Leicestershire, UK. Deuterated solvents and tetramethylsilane (TMS) were obtained from Cambridge Isotope Laboratories Inc., Andover USA. NQO1 was expressed and purified by Morvus Technology Ltd. Reactions were monitored using thin layer chromatography (TLC) on pre-coated 60-F₂₅₄ silica gel aluminium backed plates visualised by ultra-violet (UV) radiation at 254nm and 325nm using a UV GL-58 mineral light lamp or by staining with potassium permanganate (KMnO₄) solution. Column chromatography was carried out using silica gel 100-125 mesh from VWR international. The ¹H and ¹³C nuclear magnetic resonance (NMR) spectra were recorded on a Bruker Avance 300MHz or Varian 400MHz NMR spectrometers. Chemical shifts are reported as δ parts per million (ppm) downfield of TMS for samples run in deuterated chloroform (CDCl₃) or deuterated dimethyl sulfoxide (DMSO-*d₆*). The ¹³C spectra were assigned with the aid of Distortionless Enhancement through Polarisation Transfer experiments (DEPT experiments). The following abbreviations were used: s (singlet), d (doublet), dd (doublet of doublets), t (triplet) and m (multiplet). J values measured in Hz. Electron ionisation (EI) and positive and negative chemical ionisation (CI) were recorded on a Micromass Trio 2000 spectrometer. Positive and negative electrospray ionisation (ESI) was recorded using a Micromass Tof Spec 2e ionisation spectrometer. High resolution spectra (HRMS) were recorded on a Thermo Finnigan MAT95XP spectrometer. Infrared spectroscopy was recorded using Jasco FT/IR-4100 running spectra manager. All peaks were reported in wavelength cm⁻¹. Melting points (MP) were determined in open glass capillary tubes on a GallenKamp MPD.350.BM2.5 apparatus and remained uncorrected. Ultra-violet (UV) spectra were recorded on a Cary 100 UV spectrometer running Cary Win UV software using pathlength quartz cuvettes (2 opposing frosted sides), 1cm path length. Fluorescence spectra for the enzyme reactions were recorded on a Cary Eclipse fluorimeter using 4-sided quartz thermostatted cuvettes, 1cm path length. Slit values of 2.5, 5 and 10 were used depending on intensity generated, with an automatic shutter-on function being used to minimise photo-bleaching. The fluorescence emission and excitation spectra for chemical reductions were recorded in 4-sided quartz thermostatted cuvettes using a Shimadzu RF-5301PC spectrofluorophotometer. The light source was supplied *via* a 150w Xenon bulb. Data were processed using Shimadzu Rf-5301PC software. ThermoFisher Accela U-HPLC (Ultra-High Pressure Liquid Chromatography) System and separation was achieved using a C18 reverse-phase column (dimensions: 50x2.1mm; particle size 1.9 micron). The data was analyzed using a PDA (Photo Diode Array) system and processed using ChromQuest software (version 4.2). All solvents were used without further purification. In reactions, solutions were dried with MgSO₄. Solvents were evaporated under reduced pressure.
Magnetisable particles coated with anti-Ber-EP4 antibodies were obtained from (Invitrogen Dynal AS, Oslo, Norway.).

*Abbreviations*: DCC is dicyclohexylcarbodiimide; DMAP is 4-dimethylaminopyridine; DCM is dichloromethane; DCE is dichloroethane; DMSO is Dimethyl sulfoxide; PBS is Phosphate buffered saline.

### Example 1 - Lactones of General formula (IV), Y is O

### A. 6-Hydroxy-4,4,5,7,8-pentamethyl-1-benzopyran-2-one (R₁=R₂=R₃=R₄=R₅=Me)

2,3,5-Trimethyl-1,4-hydroquinone (5.0g, 32.9 mmol) and methyl 3-3-dimethylacrylate (4.31g, 4.94ml, 37.8mmol) were added to methanesulfonic acid (50ml). The mixture was stirred at 70°C for 3 hours and then quenched with H₂O (200ml) and extracted with ethyl acetate (3x100mL). The organic layer was then washed with H₂O (100mL), aqueous saturated NaHCO₃ (100mL) and saturated brine (100mL). The organic layer was then dried over MgSO₄ and condensed *in vacuo* to give a solid. The residue was recrystallised from hexane-chloroform (3:1) to give the lactone (5.4g, 70.2%) as colourless crystals. MP:182-184°C, lit 186-187°C (Borchardt and Cohen, 1972b). ¹H NMR (CDCl₃): δ 4.73 (1H, s, O**H**), 2.55 (2H, s, C**H**₂), 2.36 (3H, s, ArC**H**₃), 2.22 (3H, s, ArC**H**₃), 2.19 (3H, s, ArC**H**₃), 1.45 (6H, s, 2xgem-C**H**₃). ¹³C NMR (CDCl₃): δ 169.3 (**C**=O), 148.7, 142.3, 127.6, 123.4, 120.9, 119.5 (6xAr**C**), 45.7 (**C**H₂), 34.6 (**C**(CH₃)₂), 27.5 (2x gem-**C**H₃), 14.6, 12.4, 12.1 (3x Ar**C**H₃). MS: CI 235, (M+1)⁺ 33%; EI 234, (M)⁺ 100%.

### B. 6-Hydroxy-4,4,7,8-tetramethyl-1-benzopyran-2-one (R₁=R₂=R₁=R₅=Me; R₃=H)

2,3-Dimethyl-1,4-hydroquinone (5.03g, 36.2mmol) and methyl 3-3-dimethylacrylate (5.68mL, 4.96g, 43.5mmol) were added to methanesulfonic acid (50ml). The mixture was stirred at 90°C for 5 hours and then quenched with H₂O (200mL) and extracted into ethyl acetate (3x200mL). The organic layer was then washed with water (100mL), saturated NaHCO₃ (100mL) and saturated brine (100mL). The organic layer was then dried over MgSO₄ and condensed *in vacuo*. The residue was recrystallised from hexane-chloroform (3:1) to give the lactone as colourless crystals (3.40g, 42.7%). MP: 145-147°C, lit: 146-148°C (Yenes and Messeguer, 1999). ¹H NMR (CDCl₃): δ 6.63 (1H, s, Ar**H**), 5.21 (1H, s, O**H**), 2.58 (2H, s, C**H**₂), 2.23 (3H, s, C**H**₃), 2.16 (3H, s, C**H**₃), 1.30 (6H, s, 2x gem-C**H**₃); ¹³C NMR (CDCl₃): δ 169.4 (**C**=O), 150.3, 142.6, 126.5, 126.3, 122.7, 107.8 (6xAr**C**), 43.6 (**C**H₂), 33.1 (**C**(CH₃)₂), 27.6 (2x gem-**C**H₃), 12.3, 12.0 (2x Ar**C**H₃); MS: CI 221, (M+1)⁺ 44.7%; EI 220, (M)⁺ 100%.

### C. 6-Hydroxy-5,7,8-trimethyl-1-benzopyran-2-one (R₁=R₂=R₃=Me; R₄=R₅=H)

2,3,6,-Trimethylhydroquinone (5g, 32.9mmol) and methyl 3-3-dimethylacrylate (3.39g, 3.55mL, 39.5mmol) were added to methanesulfonic acid (50ml). The mixture was stirred at 90°C for 2 hours and then quenched with H₂O (200mL) and extracted with ethyl acetate (3x200mL). The organic layer was then washed with H₂O (100mL), saturated aqueous NaHCO₃ (100mL) and saturated aqueous brine (100mL). The organic layer was dried over MgSO₄ and condensed *in vacuo*. The residue was recrystallised from hexane-chloroform (3:1) to give the lactone (4.21g, 62.1%) as colourless crystals. MP: 169-171°C. ¹H NMR (CDCl₃): δ 4.65 (1H, s, O**H**), 2.91 (2H, t, C**H**₂ J=7.2), 2.71 (2H, t, C**H**₂ J=7.4), 2.21 (3H, s, C**H**₃), 2.19 (3H, s, C**H**₃), 2.18 (3H, s, C**H**₃); ¹³C NMR (CDCl₃): δ 169.5 (**C**=O), 148.2, 144.2, 122.9, 121.8, 119.3, 118.0, (6x**C**Ar), 29.1 (3**C**H₂), 21.3 (4**C**H₂), 12.2, 12.1, 11.8 (3xAr**C**H₃); CI 207, (M+1)⁺ 25%; EI 206, (M)⁺ 100%.

### Example 2 - Quinone Acids of General formula (II), Y is O

### A. 3-(3',6'-Dioxo-2',4',5'-trimethylcyclohexa-1',4'-diene)-3,3-dimethylpropanoic acid (R₁=R₂=R₃=R₄=R₅=Me)

6-Hydroxy-4,4,5,7,8-pentamethyl-1-benzopyran-2-one (3.7g, 15.8 mmol) was suspended in acetonitrile (aq., 15%v/v, 200ml). A solution of NBS (3.8g, 21.3 mmol) in acetonitrile (aq., 40%v/v, 60ml), was added dropwise over a period of 1 hour to the suspension. The mixture was stirred for a further 30 minutes, then diluted with H₂O (330mL) and extracted with diethyl ether (3x75mL). The combined organic phase was washed with H₂O (2x100mL) and brine (100mL), dried over MgSO₄, and then condensed at the pump to give a solid. The solid was recrystallised from ethyl acetate to afford the quinone acid as yellow crystals (1.9g, 48.1%). MP: 97-99°C, lit: 101-103°C (Borchardt and Cohen, 1973c). ¹H NMR (CDCl₃): δ 11.04 (1H, brs, O**H**), 3.06 (2H, s, C**H***₂*), 2.10, (3H, s, ArC**H***₃*), 1.95 (6H, s, 2x C**H***₃*), 1.43 (6H, s, 2x gem-C**H***₃*); ¹³C NMR (CDCl₃): δ 190.9, 187.4 (2x quinone **C**=O),178.9 (**C**OOH), 152.0, 143.0, 139.0, 138.4 (4x ring**C**), 47.3 (**C**H₂), 37.9 (**C**(CH3)₂), 28.8 (2x gem-**C**H₃), 14.3, 12.5, 12.1(3x Ar**C**H₃); MS: CI 251, (M+1)⁺ 100%; EI 250, (M)⁺ 5%.

### B. 3-(3',6'-Dioxo-4',5'-dimethylcyclohexa-1',4'-diene)-3,3-dimethylpropanoic acid (R₁=R₂=R₄=R₅=Me; R₃=H)

6-Hydroxy-4,4,7,8-tetramethyl-1-benzopyran-2-one (2.0g, 9.1mmol) was suspended in acetonitrile (aq., 15%, 110mL). A solution of NBS (2.18g, 12.3mmol) in acetonitrile (aq., 40%, 35mL), was added dropwise over 1 hour to the stirred lactone suspension. The mixture was stirred for a further 2 hours, then diluted with H₂O (180mL) and extracted into diethyl ether (3x45mL). The combined organic extracts were washed with H₂O (120mL), saturated brine (100mL) and dried over MgSO₄. The yellow organic solution was evaporated to dryness and recrystallised from ethyl acetate to yield the quinone acid as yellow crystals (1.3g, 60.5%) MP: 92-94°C. ¹H NMR (CDCl₃): δ 10.37 (1H, brs, O**H**), 6.53 (1H, s, 2-**H**), 2.94 (2H, s, C**H**₂), 2.00 (6H, s, 2xC**H**₃), 1.32 (6H, s, 2x gem-C**H**₃); ¹³C NMR (CDCl₃): δ 187.9, 187.5 (quinone **C**=O), 177.3 (**C**OOH), 152.9, 142.4, 139.7, 132.2 (4x ring**C**), 44.8 (**C**H₂), 36.8 (**C**(CH₃)₂), 28.1 (2x gem**C**-H₃), 12.6, 11.9 (2xAr-**C**H₃). MS: CI 237, (M+1)⁺ 22.4%, 254 (M+NH₄) 100% ; EI 237, (M+1)⁺ 55.3%.

### C. 3-(3',6'-Dioxo-2',4',5'-trimethylcyclohexa-1',4'-diene)propanoic acid (R₁=R₂=R₃=Me; R₄=R₅=H)

The appropriate lactone (1.5g, 7.3mmol) was suspended in acetonitrile (aq., 15%, 90mL). A solution of NBS (1.72g, 9.83mmol) in acetonitrile (aq., 40%, 30mL), was added dropwise over 1 hour to the stirred lactone suspension. The mixture was stirred for a further 1 hour, then diluted with H₂O (220mL) and extracted with diethyl ether (3x75mL). The combined organic extracts were washed with H₂O (150mL), dried over MgSO₄, condensed *in vacuo* and recrystallised from ethyl acetate to yield the quinone acid as a yellow compound (1.19g, 73.5%). MP: 113-115°C. ¹H NMR (CDCl₃): δ 11.00 (1H, br, O**H**), 2.82 (2H, t, C**H**₂ J=7.7), 2.52 (2H, t, C**H**₂ J=7.7), 2.11 (3H, s, C**H**₃), 2.02 (6H, s, 2xC**H**₃); ¹³C NMR (CDCl₃): δ 187.5, 186.9 (quinone **C**=O), 178.5 (**C**OOH), 141.8, 141.6, 140.8, 140.6 (4x ring**C**), 32.6 (2**C**H₂), 22.1 (3**C**H₂), 12.4, 12.3, 12.3 (3xAr-**C**H₃). MS: CI 223, (M+1)⁺ 100%; EI 222, (M)⁺ 19.7%.

### Example 3- Latent fluorogenic substrates of General formula (I), Y is O

### A. 2-Oxo-2H-1-benzopyran-7-yl-3-methyl-3-(2,4,5-trimetlyl-3,6-dioxo-cyclohexa-1,4-dienyl)-butanoate (R₁ = R₂=R₃=R₄=R₅=Me)(Compoud A)

A mixture of 3-(3',6'-dioxo-2',4',5'-trimethylcyclohexa-1',4'-diene)-3,3-dimethylpropanoic acid (400mg, 1.7mmol), DCC (415mg, 2.0 mmol) and DMAP (21mg, 0.2mmol) were suspended in dry DCM (10ml). The suspension was stirred for 30 minutes. 7-Hydroxycoumarin (317mg, 2.0mmol) was added and the mixture was stirred for a further 24 hours. The resulting suspension was filtered and the filtrate was evaporated. The formed suspension was filtered, evaporated and redissolved in ethyl acetate and filtered. The organic extract purified by column chromatography (1:3, ethyl acetate-hexane) to afford the product: 330mg (50.0%) as a yellow solid. MP: 128-130°C. ¹H NMR (CDCl₃): δ 7.68 (1H, d, Ar**H**, J=9.6), 7.46 (1H, d, Ar**H**, J=8.4), 7.02 (1H, d, Ar**H**, J=2.1), 6.94 (1H, d,d, Ar**H**, J=2.1) 6.39 (1H, d, Ar**H**, J=9.6), 3.29 (2H, s, C**H**₂), 2.19 (3H, s, C**H**₃), 1.94 (6H, s, 2xC**H**₃), 1.53 (6H, s, 2x gem-C**H**₃). ¹³C NMR (CDCl₃): δ 190.8, 187.3 (2x quinone **C**=O), 170.8 (**C**=OO), 160.3 (**C**=O coumarin), 154.7, 152.9, 151.4, 142.8, 142.7, 139.5, 138.9, 128.6, 118.3, 116.7, 116.2, 110.4 (12x ring**C**), 47.7 (**C**H₂), 39.0 (**C**(CH₃)₂), 29.9 (2x gem-**C**H₃), 14.5, 12.7, 12.2 (3x**C**H₃). MS: ES+ve 417.1 (M+Na) 100%.

### B. 2-Oxo-2H-1-benzopyran-7-yl-3-(4,5-dimethyl-3,6-dioxo-cyclohexa-1,4-dienyl)-3-metlyl-butanoate (R₁ = R₂=R₄=R₅=Me; R₃=H)(Compound B)

To a suspension of 3-(3',6'-dioxo-4',5'-dimethylcyclohexa-1',4'-diene)-3,3-dimethylpropanoic acid (400mg, 1.7mmol) in dry DCM (10mL) was added DCC (4.18mg, 2.0mmol) and DMAP (21mg, 0.2mmol). The mixture stirred for 30 minutes and then 7-hydroxycoumarin (329mg, 2.0mmol) was added. The mixture was then stirred for 16 hours. The resultant mixture was filtered and condensed *in vacuo.* The resultant solid was dissolved in ethyl acetate and filtered. The filtrate was condensed and redissolved in ethyl acetate and filtered, the resultant filtrate was again reduced *in vacuo* and purified by column chromatography (1:3 ethyl acetate-hexane) to yield the product as a yellow solid 285mg (44.4%). MP: 108-110°C. ¹H NMR (CDCl₃): δ 7.66, (1H, d, Ar**H**, J=9.6), 7.44 (1H, d, Ar**H**, J=8.4), 7.00 (1H, d, Ar**H**, J=1.8), 6.93 (1H, d,d, Ar**H**, J=2.1, 2.1), 6.59 (1H, s, Ar**H**), 6.38 (1H, d, Ar**H**, J=9.6), 3.22 (2H, s, C**H**₂ ), 2.02 (3H, s, C**H**₃), 1.94 (3H, s, C**H**₃), 1.42 (6H, s, 2xgem-C**H**₃). ¹³C NMR (CDCl₃): δ188.0 187.0 (2x**C**=O), 170.1 (**C**=OO), 160.7 (**C**=O coumarin), 154.9, 153.2, 153.1, 143.3, 142.7, 140.3, 132.6, 129.0, 118.7, 117.1, 116.4, 110.7 (12x ring**C**), 45.4 (**C**H₂), 37.7 (**C**(CH₃)₂), 28.6 (2x gem-**C**H₃), 13.0, 12.3 (2x**C**H₃). MS: ES+ 403.1 (M+Na).

### C. 2-Oxo-2H-1-benzopyran-7-yl-3-(2,4,5-trimethyl-3,6-dioxo-cyclohexa-1,4-dienyl)-propartoate (R₁=R₂= R₃=Me; R₄=R₅=H)(Compound C)

To a solution of DCC (557mg, 2.7mmol) and DMAP (27mg, 0.2mmol) in dry DCM (10ml) was added to 3-(3',6'-dioxo-2',4',5'-trimethylcyclohexa-1',4'-diene) propanoic acid (500mg, 2.3mmol) and the suspension was stirred for 30 minutes. To the mixture was added 7-hydroxycoumarin (437mg, 2.7mmol) and stirring was continued for 16 hours at room temperature. The resultant mixture was filtered and condensed *in vacuo.* The resultant solid was dissolved in ethyl acetate and filtered. The filtrate was condensed and redissolved in ethyl acetate and filtered. The resultant filtrate was again reduced *in vacuo* and purified by column chromatography (eluting with 1:3 ethyl acetate:hexane) to yield the product as a yellow solid 285mg (34.6% yield). ¹H NMR (CDCl₃): δ 7.69 (1H, d, Ar**H** J=9.6), 7.49 (1H, d, Ar**H** J=8.4), 7.13 (1H, d, Ar**H** J=2.1), 7.06, 7.04 (1H, d,d, ArH J=2.1), 6.40 (1H, d, ArH J=9.6), 2.94 (2H, t, CH₂ J=7.5), 2.76 (2H, t, C**H**₂ J=7.5), 2.11 (3H, s, C**H**₃), 2.03 (3H, s, C**H**₃).

### D. 4-Methyl-2-oxo-2H-chromen-7-yl-3-methyl-3-(2,4,5-trimethyl-3,6-dioxocyclohexa-1,4-dienyl)butanoate (R₁= R₂=R₃=R₄=R₅=Me) [MTL8-252]; (Compourtd D)

A mixture of 3-(3',6'-dioxo-2',4',5'-trimethylcyclohexa-1',4'-diene)-3,3-dimethylpropanoic acid (800mg, 3.4mmol), DCC (830mg, 4.0 mmol) and DMAP (42mg, 0.4mmol) were suspended in dry DCE (20ml). The suspension was stirred for 30 minutes. 4-metylumberlliferone (634mg, 3.6mmol) was added and the mixture was stirred for a further 24 hours. The resulting suspension was filtered and the filtrate was evaporated. The formed suspension was filtered, evaporated and redissolved in ethyl acetate and filtered. The organic extract was evaporated and recrystallised from ethyl acetate to afford the product as yellow crystals (0.96g, 69%).¹H NMR (CDCl₃): δ 7.58 (1H, d, Ar**H**, J=5Hz), 7.02 (1H, d, Ar**H**, J=3Hz), 6.98 (1H, d, Ar**H**, J=3Hz), 6.96 (1H, d, Ar**H**, J=3Hz), 3.29 (2H, s, C**H**₂), 2.41 (3H, s, C**H**₃), 2.18 (3H, s, C**H**₃), 1.94 (3H, s, C**H**₃), 1.53 (6H, s, 2xC**H**₃). ¹³C NMR (CDCl₃): δ 190.8, 187.3 (2x quinone **C**=O), 170.8 (**C**=OO), 160.4 (**C**=0 coumarin), 154.1, 152.8, 151.8, 151.5, 142.7, 139.7, 138.8, 125.4, 117.9, 117.8, 114.6, 110.4 (12x ring**C**), 47.7 (**C**H₂), 38.4 (**C**(CH₃)₂), 29.0 (2x gem-**C**H₃), 18.7 (**C**H₃), 14.4, 12.6, 12.1 (3x**C**H₃).

The following amido substrates were synthesized as models of the compounds of general formula (I) in which X is NR⁸.

### Example 4 - Amido substrates

### A. N-metlyl-N-phenyl-[3-(2,4,5-trimetlyl-3,6-dioxocyclohexa-1,4-dienyl)-3-methyl]butanamide

To the appropriate quinone acid (400mg, 1.6mmol) was added DCC (371mg, 1.8mmol) and DMAP (22mg, 0.2mmol) in dry DCM (10ml). The mixture was stirred for 30 mins, after which time N-methylaniline (192.6mg, 1.8mmol) was added. The mixture was then stirred for 16 hours. The resultant suspension was filtered and washed with HCl (aq., 0.1M, 5ml), reduced *in vacuo* and purified by column chromatography (eluting with 3:1 ethyl acetate:hexane) to yield a yellow solid of the product: 390mg (71.9%). MP: 92-94°C. ¹H NMR (CDCl₃): δ 7.45 (2H, t, meta **H**, J=6.9), 7.36 (1H, t, para **H** J=6.8), 7.20 (2H, d, ortho **H** J= 7.2), 3.17 (3H, s, NC**H**₃), 2.75 (2H, s, C**H**₂), 2.10 (3H, s, C**H**₃), 2.01 (3H, s, C**H**₃), 1.97 (3H, s, C**H**₃), 1.30 (6H, s, 2xgem-C**H**₃). ¹³C NMR (CDCl₃): δ 191.2 (Quinone **C**=O), 187.7 (Quinone **C**=O), 172.1 (**C**=O), 154.8, 144.0, 143.6, 137.7, 136.2 (5x ring**C**), 129.8 (meta **C**), 127.8 (para **C**), 127.5 (ortho **C**), 47.6 (**C**H₂), 38.0 (**C**(CH₃)₂), 37.1 (N**C**H₃), 28.4 (2x gem-**C**H₃), 14.1, 12.7, 12.1 (3x **C**H₃). MS: ES+ 362.2 (M+Na).

### B. N-methyl-N-phenyl-[3-(4,5-Dimethyl-3,6-dioxocyclohexa-1,4-dienyl)-3-methyl]butartamide

To a solution of DCC (371mg, 1.80mmol), and DMAP (22mg, 0.180mmol) in dry 10ml DCM, 3-(3',6'-dioxo-4',5'-dimethylcyclohexa-1',4'-diene)-3,3-dimethylpropanoic acid (400mg, 1.69mmol) was added. The mixture was stirred for 30 minutes after which time N-methylaniline (181.8mg, 1.80mmol) was added. The mixture was then stirred for 16 hours. The mixture was filtered and washed with HCl (aq., 0.1M, 5ml) and reduced *in vacuo* and purified by column chromatography (eluting with 3:1 ethyl acetate:hexane) gave a yellow solid of the product 330mg (60.4%). MP: 110-112°C. ¹H NMR (CDCl₃): δ 7.44 (2H, t, meta **H** J=7.4), 7.36 (1H, t, para **H** J= 7.2), 7.16 (2H, d, meta **H** J=7.5), 6.49 (1H, s, quinone Ar**H**), 3.16 (3H, s, NC**H**₃), 2.02 (3H, s, C**H**₃), 1.99 (3H, s, C**H**₃), 1.16 (6H, s, 2x gem-C**H**₃). ¹³C NMR (CDCl₃): δ 188.2 (Quinone **C**=O), 188.0 (Quinone **C**=O), 171.3 (**C**=O), 155.5, 144.1, 142.3, 139.6, 130.4 (5x ring**C**), 129.8 (2x meta **C**), 127.8 (para **C**), 127.5 (2x ortho **C**), 44.4 (*C*H₂), 37.4 (**C**(CH₃)₂), 37.2 (N**C**H₃), 28.5 (2x gem-**C**H₃), 12.7, 11.9 (2x Quinone **C**H₃). MS: ES+ 348.1 (M+Na).

### C. N-Metlyl-N-phenyl-3-(2,4,5-trimetlyl-3,6-dioxocyclohexa-1,4-diertyl)-propanamide (R₁=R₂=R₃=Me; R₄=R₅=H)

To the appropriate quinone acid (64) (400mg, 1.8mmol) was added DCC (371mg, 1.8mmol) and DMAP (22mg, 0.2mmol) in dry DCM (10ml) and stirred for 30 minutes. To the stirring mixture was added *N*-methylaniline (192.6mg, 1.8mmol). The mixture was then stirred for 16 hours. The resultant mixture was filtered and washed with HCl (aq., 0.1M, 5ml) and reduced *in vacuo* and purified by column chromatography (eluting with 3:1 ethyl acetate:hexane) to yield a yellow solid of the product: 285mg (50.9%). MP: 69-71°C. ¹H NMR (CDCl₃): δ 7.40 (2H, t, meta **H** J=7.4), 7.32 (1H, t, para **H** J=7.2), 7.17 (2H, d, ortho **H** J=7.5), 3.26 (3H, s, NC**H**₃), 2.75 (2H, t, C**H**₂ J=7.8), 2.19 (2H, t, C**H**₂, J=7.8) 2.00 (3H, s, 2xC**H**₃), 1.94 (3H, s, C**H**₃). ¹³C NMR (CDCl₃): δ 187.6 (Quinone **C**=O), 186.8 (Quinone **C**=O), 171.7 (**C**=O), 143.8, 142.9, 141.0, 140.4 (5x ring **C**), 129.8 (2x Meta **C**), 127.9 (Para **C**), 127.3 (2x Ortho **C**), 37.4 (N**C**H₃), 32.8, 23.0 (2x**C**H₂), 12.4, 12.3, 12.1 (3x**C**H₃). MS: ES+ 334.2 (M+Na).

### D. N-phenyl[3-Methyl-3-(2,4,5-trimethyl-3,6-dioxocyclohexa-1,4-dienyl)]butartamide (R₁=R₂=R₃=R₄=R₅=Me)

To the appropriate quinone acid (400mg, 1.6mmol) was added DCC (371mg, 1.8mmol) and DMAP (22mg, 0.2mmol) in dry 10ml DCM and stirred for 30 minutes. To the stirring mixture was added aniline (148.8mg, 1.6mmol). The mixture was then stirred for 16 hours at room temperature. The resultant mixture was filtered and washed with HCl (aq., 0.1M, 5ml) and reduced *in vacuo* and purified by column chromatography (eluting with 3:1 ethyl acetate:hexane) to yield a yellow solid product: 410mg (78.8%). MP: 152-154°C. ¹H NMR (CDCl₃): δ 7.40 (2H, d, ortho **H** J=8.1), 7.28 (2H, t, meta **H** J= 7.7), 7.13 (1H, s, N**H**) 7.07 (1H, t, para **H** J=7.4), 3.02 (2H, s, C**H**₂), 2.15 (3H, s, C**H**₃), 1.95 (6H, s, 2xC**H**₃), 1.50 (6H, s, 2xgem-C**H**₃). ¹³C NMR (CDCl₃): δ 191.6 (Quinone **C**=O), 187.5 (Quinone **C**=O), 170.2 (**C**=O), 153.0 (Quinone alkene **C**), 143.3 (Quinone alkene **C**), 138.3 (Quinone alkene **C**), 138.2 (aniline ipso **C**), 137.6 (Quinone alkene **C**), 129.0 (Meta **C**), 124.3 (Para **C**), 119.8 (Ortho **C**), 50.5 (**C**H₂), 38.4 (**C**(CH₃)₂), 29.1 (2xgem-**C**H₃), 14.2 (Quinone **C**H₃), 12.7 (Quinone **C**H₃), 12.2 (Quinone **C**H₃). MS: ES+ 348.1 (M+Na).

### E. N-phenyl-[3-(4,5-Dimethyl-3,6-dioxocyclohexa-1,4-dienyl)-3-methyl] butanamide (R₁=R₂=R₄=R₅=Me; R₃=H)

To the appropriate quinone acid (400mg, 1.69mmol) was added DCC (371mg, 1.80mmol) and DMAP (22mg, 0.180mmol) in dry DCM (10ml) and stirred for 30 minutes. To the stirring mixture was added aniline (157.2mg, 1.69mmol). The mixture was then stirred for 16 hours at room temperature. The resultant mixture was filtered and washed with HCl (aq., 0.1M, 2ml) and reduced *in vacuo* and purified by column chromatography (eluting with 3:1 ethyl acetate:hexane) to yield a yellow solid (86) 390mg (73.9%). MP: 131-133°C. ¹H NMR (CDCl₃): δ 7.37 (2H, d, ortho **H** J=7.5), 7.27 (2H, t, meta **H**, J=7.8), 7.07 (2H, t, para **H**, J=7.4) 7.05 (N**H**, D₂O exchange), 6.60 (1H, s, quinone **H**), 2.94 (2H, s, C**H**₂), 2.02 (3H, s, C**H**₃), 2.00 (3H, s, C**H**₃), 1.38 (6H, s, 2xgemC**H**₃). ¹³C NMR (CDCl₃): δ 188.4 (quinone carbonyl **C**=O), 187.9 (quinone carbonyl **C**=O), 169.1 (amide carbonyl **C**=O), 153.5 (quinone **C**), 142.3 (quinone **C**), 140.0 (quinone **C**), 137.4 (aniline ipso **C**), 132.0 (quinone **C**), 129.0 (meta **C**), 124.4 (para **C**), 119.8 (ortho **C**), 48.3 (**C**H₂), 37.6 (**C**(CH₃)₂), 28.5 (2xgem**C**H₃), 12.6 (quinone **C**H₃), 11.9 (quinone **C**H₃). MS: ES+ 334.1 (M+Na).

### F. N-Phenyl-3-(2,4,5-trimethyl-3,6-dioxocyclohexa-1,4-dienyl)-propanamide (R₁=R₂=R₃=Me; R₄=R₅=H)

To the appropriate quinone acid (400mg, 1.80mmol) was added DCC (371mg, 1.80mmol) and DMAP (22mg, 0.180mmol) in dry DCM (10ml) and stirred for 30 minutes. To the stirring mixture was added aniline (167.4mg, 1.80mmol). The mixture was then stirred for 16 hours at room temperature. The resultant mixture was filtered and washed with HCl (aq., 0.1M, 2ml) and reduced *in vacuo* and purified by column chromatography (eluting with 3:1 ethyl acetate:hexane) to yield a yellow solid of the product: 360mg (67.2%). MP: 135-137 °C. ¹H NMR (CDCl₃): δ 8.09 (1H, s, N**H**), 7.51 (2H, d, ortho **H** J=7.8), 7.30 (2H, t, meta **H** J=8.0), 7.08 (1H, t, para **H** J= 7.4), 2.89 (2H, t, C**H**₂ J=7.7), 2.50 (2H, t, C**H**₂, J=7.8), 2.08 (3H, s, C**H**₃), 2.00 (6H, s, 2xC**H**₃). ¹³C NMR (CDCl₃): δ 187.4, 187.4 (2x**C**=Oquinone), 170.0 (**C**=O), 142.2, 141.7, 141.0, 140.4, 137.8 (5x ring**C**), 129.0 (meta **C**), 124.3 (para **C**), 119.8 (ortho **C**), 36.2 (**C**H₂), 23.0 (**C**H₂), 12.5, 12.3 (3x**C**H₃). MS: ES+ 320.2 (M+Na) 100%.

### Example 5 - Solubility and Purity

NMP Solubility: A solution of the product of Example 3D (MTL8-252) was prepared in NMP and further diluted to 0.1M in NMP. The purity of the compound was assessed via uplc using a 1-99% Acetonitrile gradient. Injection volumes of 0.5µl, 1µl, 2µl, 3µl and 5µl were used. Purity was determined to be almost 100% based on uv data at 265nm. The FL Plus settings were (high PMT voltage, λex 410nm and λem 550nm, off peak). Only a single peak with retention time of approx 4.3 min was detected. MTL8-252 was equally very soluble in DMSO.

Aqueous solutions of NMP and DMSO stocks in 10mM sodium phosphate buffer, pH 10 resulted in a slightly cloudy solution that did not pellet when spun at 13000rpm in a bench microfuge. However, the peak area from UV hplc trace did not appear to have been altered (compared to that of NMP diluted sample). Filtration using a 0.2micron membrane resulted in a clear solution but no peak was detected on hplc, suggesting that the material was retained on the nylon filter. A similar result was obtained for the diluted DMSO stock. Subsequent experiments utilised 0.1M diluted stock in 10mM phosphate buffer, pH 7.0 and injected directly onto the uplc column.

### Example 6 - Stability of MTL8-252 in NMP and sodium phosphate Buffer

Stability of 0.1M MTL8-252 in NMP was assessed over a 2-hour period at 37°C. The compound appeared stable as evidenced by the fact that the uv absorbance peak area remained substantially unchanged. No detectable release of 4-methylumbelliferone (4-MU) was observed. A similar experiment was carried out with DMSO stock diluted in 10mM sodium phosphate buffer. The result (see Figure 2) shows MTL8-252 is significantly stable in this solution for at least 1hr.

### Example 7 - Evaluation of MTL8-252 Activity with human NQOI

Initial HPLC was carried out on samples containing MTL8-252 (0.1mM), NADH (0.5mM) and 0.5µg/ml hNQO1 in phosphate buffer (10mM, pH 7) using a 1-99% acetonitrile gradient and a µl injection volume. Temperature was maintained at 37°C. The results are shown in Table 1 below.

**Table 1 - Summary Data (Initial rates)**

| Rate of decrease in MTL8-252 concentration (µM/min) (n =4) | Rate of formation of 4-MU (µM/min) by FL (n=2) | Rate of formation of 4-MU (µM/min) @ 210nm (n =2) | Rate of formation of Lactone (µM/min) (n = 2) |
|---|---|---|---|
| **5.9, 6.21, 8.41, 9.05** | **13.51, 6.90** | **11.48, 6.0** | **9.84, 7.61** |

Reduction of the parent compound was rapid as most of it had disappeared by the second injection. An enzyme concentration of 0.2µg/ml hNQO1 was found to be suitable for determining the initial rate of MTL8-252 reduction. No significant reduction in the area of the peak was observed in the presence of either MTL8-252 alone or with NADH alone (see Figure 3). The rate of reduction in MTL8-252 concentration was calculated at 5.9µM/min and when the concentration of the enzyme was halved the rate reduced to 1.5µM/min.

This finding demonstrates that MTL8-252 is a substrate for hNQO1. There was an increase in 4-MU peak area when monitored at 325nm that matched the loss of substrate. This was confirmed by an increase in 4-MU fluorescence peak. The presence of NADH did not interfere with the FL spectra.

The cut off point for the UV data was initially set between 220-800nm and when the lower wavelength was reduced to 180nm, the lactone, 4-MU and MTL8-252 were quite evident despite minimal baseline noise interference.

By altering the settings of the FL Plus detector (Low PMT voltage, λex 410nm , λem 550nm) and calibrating for 4-MU measurements by establishing a calibration graph it can be seen that there was a linear response to 4-MU up to at least 200µM. The maximum concentration used in incubation experiments is 100µM, final.

Following calibration of the instrument, a repeat experiment of the activation of MTL8-252 by hNQO1 was carried out resulting in calculated rates of loss of MTL8-252 and formation of 4-MU (see Figure 4). Rate loss of MTL8-252 was 8.41uM/min and that corresponding to 4-MU formation determined by fluorescence was 13.51uM/min.

Replots of UV data at 210nm, showed initial rates of formation of 4-MU and lactone were 11.48uM/min and 9.84uM/min, respectively (see Figure 5).

### Example 8 - Activity of 4-MU and Lactone with hNQO1

This experiment was performed to ensure the products of activation were not further metabolised by hNQO1. HPLC was carried out on samples containing 4-MU and Lactone (0.1mM), NADH (0.5mM) and hNQO1 (0.2µg) in phosphate buffer (10mM, pH 7) using a 1-99% acetonitrile gradient and a 2µl injection volume. Temperature was maintained at 37°C. The results show both by products released following activation of MTL8-252 were not substrates of hNQO1 (see Figure 6).

### Example 9 - MTL8-252 Incubation with cultured cells in vitro

### A. Luminescence Spectrophotometric Determination

Human NQO1 expressing cells (HDT7) and null expressing cells (F179) were cultured in Eagle's MEM medium enriched with non-essential amino acids and 10% FBS. Cells were harvested in exponential growing phase and suspended in cold PBS @ 5x 10⁶ cells/ml. 100ul of cell suspension (5x10⁵ cells) were added to 3ml PBS containing 10uM MTL8-252 at 37°C in a fluorimeter cuvette and the rate of increase in fluorescence intensity (attributed to 4-MU release), measured over 3 minutes. Control experiment contained substrate alone in PBS.
The Luminescence Spectrophotometer settings were as follows: λex= 360nm; λem= 452 @ 37°C.

Figure 7 shows a significant increase in 4-MU formation rate (at least 5-fold) following incubation with the hNQO1-expressing cells (HDT7) when compared with the null expressing cells (F179). There was an indication of an initial burst of activity but because of the low sensitivity of the instrument this could not be verified.

However, the experiment was repeated on a more sensitive FL-Plus detector linked to a uplc.

### B. Uplc Separation followed by FL-Plus Detection

Cells in exponential growth phase were harvested and resuspended in cold PBS at 5x10⁶cells/ml. 100ul of cell suspension (5x10⁵cells) were added to 1ml PBS containing 10uM MTL8-252 at 37°C and incubated for the following time periods: 0,5,30 and 60min. The reaction was stopped by spinning down cells at 13,000rpm for 30 seconds and the cell free extract injected (2ul) directly unto the uplc. Separation of metabolites was achieved using a Whatman Partisil C18 column (S/N no. 4SF03177) with a gradient of 1-99% MeCN over 10min at a flow rate of 1ml/min. FL-Plus detector settings were as follows: PMT (low), λex= 360nm; λem = 450nm. Figure 8 below shows the rate of formation of 4-MU by hNQO1 expressing cells. Initial rate measurements suggest about 100-fold increase in activity over null expressing cells. There is also confirmation of an initial burst in activity as suggested in the luminescence spectrophotometric experiment.

Replacement of PBS with cell culture medium suggests other components in the media can reduce MTL8-252, Figure 9 below. Possible source is from FBS added in media.

### Example 10 - Typical protocol for MTL8-252 Incubation with cultured cells in vitro

The following general method was adopted for the results shown in figures 10-19. A 1mM solution of fluorogenic substrate was prepared in DMSO. Urine samples or buffer samples (typically 5 - 20ml as specified in a given experiment) containing cells were centrifuged at 1200 rpm in order to pellet the cells. The supernatant was removed and the pellet washed with 10ml of PBS or culture medium as prescribed for a given experiment. The cell pellet was resuspended in the desired buffer/medium (1ml) and 10ul of the substrate solution added with mixing. Following incubation at 37°C for the desired time period (typically 3 minutes), the mixture was passed through a 0.45um syringe filter and the filtrate kept on ice until analysed by fluorimetry (100ul, 380nm ex/480nm em) or hplc (uplc) (2ul). NQO1 activity is expressed as concentration of product (4-MU) produced. Certain media exhibited higher background fluorescence than others.

### Example 11 - Assay precision

The standard method described above was performed on replicate samples of NQO1-producing HDT7 cells added into culture medium or urine. Figure 10 shows the precision of triplicate determinations of the specified number of cells added into culture medium. Figure 11 shows the precision of duplicate measurements of HDT7 cells (10⁶ cells) added into urine. Error bars are SEM and the experiment demonstrates that the cells can be measured in either culture medium or urine without any adverse effect on precision.

The method was also used to assay various cell types for NQO1 activity, namely HDT7 and F179 (NQO1-producing and null engineered cell lines respectively), human bladder cancer cell lines EJ138 and RT112 and human prostate cancer cell line PC3. The results are shown in figures 12 and 13. Similar numbers of cells were used within each experiment to permit comparison. NC represents a non-cellular negative control.

### Example 13 - Discrimination between NQO1 and NQO2 activity

Prostate carcinoma cells (as exemplified by PC3 cells) express both NQO1 and NQO2 activity. However, the latter is only activated by the presence of a co-substrate not naturally present in the cells (EP-0152R, 1-carbamoylmethyl-3-carbamoyl-1,4-dihydronicotinamide). This was demonstrated as follows:
To 440ul of PBS containing 5x10⁵ PC-3 cells pre-incubated at 37°C for 5min was added 10uM MTL8-252 fluorogenic substrate in DMSO (final, 10ul) and volume adjusted to 500ul with PBS. After incubating for a further 5min the mixture was syringe filtered as above and 2ul of supernatant analysed by uplc as described earlier. The reaction on the same number of cells was repeated with the inclusion of 100uM EP-0152R (50ul) as co-substrate during incubation. The reaction mixture was filtered and analysed as above. It is known that in the absence of EP-152R hNQO2 is inactive. Figure 14 shows that there is a 1.2-fold increase in the formation of 4-MU by addition of an hNQO2 selective co-substrate. This additional 4-MU release indicates that the assay, when supplemented with hNQO2 co-substrate, can be used to detect tumour cells that, additionally or alternatively, express the hNQO2 enzyme. In the former instance, where NQO1 and NQO2 are expressed, the supplemented assay can be used to provide a stronger assay signal and in the latter instance, where only NQO2 is expressed, the supplemented assay can be used to detect hNQO2 expressing cancers only.

### Example 14 - Relationship between number of cancer cells and assay response

Various numbers of RT112 cells were subjected to the above assay to demonstrate the quantitation capabilities of the method. The data for these tests are shown in figure 15. As one would expect in a functional assay, as the number of NQO1 cells increases so does the magnitude of the assay signal. Contemporaneously, the cells were also exposed to 4',6-diamidino-2-phenylindole (DAPI), using established protocols, to permit quantitation of the total cell number by measurement of cellular DAPI fluorescence intensity in the fluorimeter (figure 16). Thus, assessment of the data in figures 15 and 16 enables one to determine the amount of signal per cell and the number of cells in a sample producing said signal. This is particularly advantageous where the NQO1 or NQO2 activity is desired to be determined on a "per cell" basis.

### Example 15 - Enrichment/isolation/purification of cells

In certain situations it is advantageous to improve sensitivity and/or specificity of the method by enrichment/isolation/purification of cells prior to assay. This example demonstrates the ability and compatibility of the present invention with such procedures. Here, samples containing cancer cells carrying the Ber-EP4 epithelial antigen were isolated using magnetisable particles coated with antibodies to this antigen (Invitrogen). The particles were used according to the Manufacturer's instructions such that a suspension of particle/cell complexes was obtained which was processed in the standard assay protocol described above. Figure 17 shows that recovery of NQO1 activity in RT112 bladder cancer cells is approximately 60 - 70% relative to that of the total number of cells present (100 %, represented by the 0 beads bar in the figure) over a range of particle densities (illustrated by the volume of Manufacturer's bead stock solution (4 x 10⁶ particles/ml) added). Figure 18 shows the comparable results for HDT7 cells which express NQO1 but do not express human Ber-EP4 antigen. This illustrates the ability to specifically enrich the epithelial cell population, a procedure that may be desirable in instances where a sample contains a large number of cells, not all likely to be over-expressing NQO1 or NQO2, but which may increase non-specific background signal in the assay.

### Example 16 - Detection of NQO1 in clinical samples

Urine samples (20ml) were obtained from patients attending a urology clinic and subjected to the assay outlined above. Results are expressed as the ratio of 4-MU concentration (produced from the fluorogenic substrate as the result of NQO1 activity) of the samples to that of the negative assay control and correlated with the eventual clinical diagnosis (transitional cell carcinoma, or not). The open triangle represents a sample containing significant amounts of debris suggesting that the use of immunoextraction as described herein would be beneficial for increasing specificity. The open circle represents a sample from a patient subsequently diagnosed with very early stage Ta bladder cancer and it is likely that there were insufficient cells for reliable analysis of this sample. The chemiluminogenic substrates described herein are likely to be advantageous in situations where higher sensitivity of detection is required.

Table 2 shows the corresponding results for patients already diagnosed with bladder cancer but as yet untreated. Sample 89 in this series could not be assayed due to the presence of gross haematuria.

Further urine samples (20ml) were collected from patients diagnosed with prostate carcinoma. These samples were collected following digital rectal examination. Table 3 shows the results of the NQO1 assay for these samples. In both of these experiments, greater NQO1 activity was seen in those samples from patients with later stage tumours. In the method of the invention the quantitative increase in NQO1/NQO2 activity with advanced disease means the method can be used, not only to indentify cancer, but also to assess the progress of the disease by relating the quantitative nature of the assay to the various stages of the disease. Those skilled in the art will appreciate this is possible because of the sensitivity of the assay and the pathology of the disease.

**Table 2**

| **Bladder cancer samples** | | |
|---|---|---|
| Sample | Stage | Ratio of sample 4-MU/negative assay control (NC) 4-MU concentrations |
| 88 | T2 | 4.0 |
| 90 | UNK | 2.9 |
| 91 | Ta | 1.4 |
| 92 | Ta | 1.5 |
| 93 | Ta | 1.0 |
| NC | n/a | 1.0 |

| | | |
|---|---|---|
| NC is negative assay control, UNK is unknown | | |

**Table 3**

| **Prostate cancer samples** | | | | |
|---|---|---|---|---|
| Sample | PSA | Stage | Observations | Ratio of sample 4-MU/negative assay control (NC) 4-MU concentrations |
| 105 | 6.6 | T1c | Post-DRE urine | 1.2 |
| 106 | 9.2 | UNK | Post-DRE urine | 1.4 |
| 107 | 6 | T1c | Post-DRE urine | 1.4 |
| 108 | 3.7 | T2a | Post-DRE urine, previous TURP | 4.0 |
| 109 | 6 | T1c | No DRE | 1.0 |
| NC | n/a | n/a | | 1.0 |

| | | | | |
|---|---|---|---|---|
| NC is negative assay control, UNK is unknown Note: This cohort was originally believed to be all post-DRE urine collection. However, on checking records, sample 109 was obtained without DRE. Precision of measurement is typically <10% CV. | | | | |

## Claims

1. A method of determining the presence or absence, in a urine sample from a patient, of bladder or prostate cancer cells which over-express NQO1 and/or NQO2, the method comprising:
i. contacting the urine sample, or a processed derivative thereof, with a compound of general formula (I), (Ia), (Ib), (Ic), (Id), (Ie) or (If) or a salt of any thereof where applicable, wherein the urine sample contains or is suspected of containing bladder or prostate cancer cells which over-express NQO1 and/or NQO2;
ii. optionally, in the case where the bladder or prostate cancer cells over-express or are suspected of over-expressing NQO2, adding an NQO2 co-substrate to the sample; and
iii. determining the presence or absence of a compound of the formula:
z-XH;
or an ion of the formula:
z-X⁻;
wherein z and X are as defined in general formula (I), wherein presence of the compound or ion indicates the presence in the sample of cancer cells which over-express NQO1 and/or NQO2
wherein general formula (I) is: 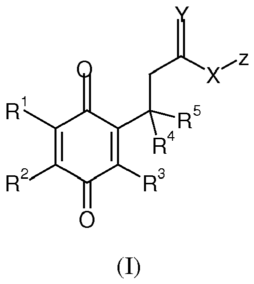
wherein R¹, R², R³, R⁴ and R⁵ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo; or
R¹ and R² together with the carbon atoms to which they are attached form a 5- or 6-membered optionally substituted aromatic, heteroaromatic, carbocyclic or heterocyclic ring system;
X is O, S or NR⁸;
R⁸ is hydrogen, or C₁-C₃ alkyl;
Y is O, S or NR⁹;
R⁹ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻;
general formula (Ia) is: 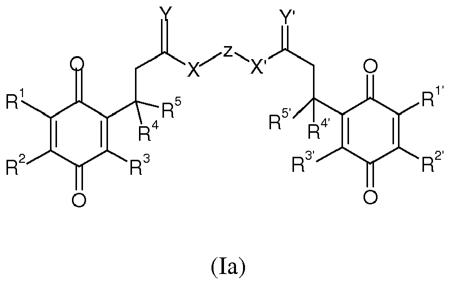
wherein R¹, R², R³, R⁴ and R⁵ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo; or
R¹ and R² together with the carbon atoms to which they are attached form a 5- or 6-membered optionally substituted aromatic, heteroaromatic, carbocyclic or heterocyclic ring system;
R^{1'}, R^{2'}, R^{3'}, R^{4'} and R^{5'} each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo; or
R^{1'} and R^{2'} together with the carbon atoms to which they are attached form a 5- or 6-membered optionally substituted aromatic, heteroaromatic, carbocyclic or heterocyclic ring system;
X is O, S or NR⁸;
R⁸ is hydrogen, or C₁-C₃ alkyl;
Y is O, S or NR⁹;
R⁹ is hydrogen, or C₁-C₃ alkyl;
X' is O, S or NR⁸;
R⁸ is hydrogen, or C₁-C₃ alkyl;
Y' is O, S or NR⁹;
R⁹ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻;
general formula (Ib) is: 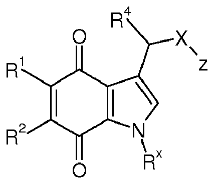
wherein R¹, R² and R⁴
each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo; or
R¹ and R² together with the carbon atoms to which they are attached form a 5- or 6-membered optionally substituted aromatic, heteroaromatic, carbocyclic or heterocyclic ring system;
R^{x} is H, or C₁-C₃ alkyl
X is O, S or NR⁸;
R⁸ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻;
general formula (Ic) is: 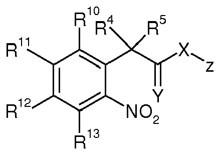
wherein R⁴, R⁵, R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo;
X is O, S or NR⁸;
R⁸ is hydrogen, or C₁-C₃ alkyl;
Y is O, S or NR⁹;
R⁹ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻;
general formula (Id) is: 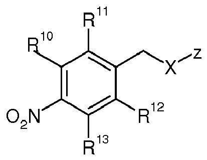 wherein:
X is O, S or NR⁸;
R⁸ is hydrogen, or C₁-C₃ alkyl;
R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo;
z is a moiety which is covalently linked to the remainder of the molecule and which, on reduction of the compound of general formula (I), is cleaved from the remainder of the molecule to form a detectable compound z-XH or ion z-X⁻;
general formula (Ie) is: 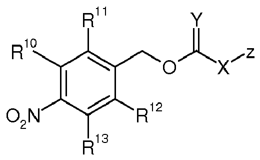 wherein
R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo;
X is O, S or NR⁸;
R⁸ is hydrogen, or C₁-C₃ alkyl;
Y is O, S or NR⁹;
R⁹ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻;
general formula (If) is: 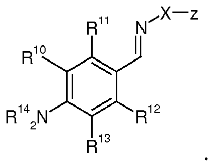 wherein
R¹⁰, R¹¹, R¹² and R¹³ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more reactive substituents;
R⁶ and R⁷ each independently represent hydrogen or C₁-C₆ alkyl optionally substituted with halo;
R¹⁴ is H or C₁-C₆ alkyl and
X is O, S or NR⁸;
R⁸ is hydrogen, or C₁-C₃ alkyl;
z is a moiety which is covalently linked to the remainder of the molecule and which, on reduction of the compound of general formula (I), is cleaved from the remainder of the molecule to form a detectable compound z-XH or ion z-X⁻.

2. The method as claimed in claim 1 wherein, in the case where the bladder or prostate cancer cells over-express or are suspected of over-expressing NQO1, adding an NQO1 co-substrate to the sample.

3. The method as claimed in claim 1 or 2 wherein z comprises a chromophore or luminophore.

4. The method as claimed in claim 3 wherein z comprises a fluorescent, phosphorescent, chemiluminescent or bioluminescent marker such that z-XH or z-X-is a fluorescent, phosphorescent, chemiluminescent or bioluminescent molecule or ion; or a modulator of emissions from a fluorescent, phosphorescent, chemiluminescent or bioluminescent molecule or ion; or a co-factor for a chemiluminescent or bioluminescent reaction.

5. The method as claimed in claim 4 wherein the optical properties of the moiety z change when it is cleaved from the remainder of the compound of general formula (I) to form the compound z-XH or the ion z-X⁻.

6. The method as claimed in claim 5, wherein the change in optical properties of moiety z comprise a detectable change in the wavelength of emitted light, the removal of a quenching effect exerted by the quinone moiety of general formula (I) or, in the case of co-factors, a modulation of their activity.

7. The method as claimed in claim 1 wherein the moiety z comprises a detectable particle, for example a coloured latex microparticle, gold nanoparticle or magnetic particle.

8. The method as claimed in claim 1 or claim 7 wherein the moiety z comprises a binding portion which selectively binds a capture moiety wherein capture moiety comprises a binding partner for the moiety z.

9. The method as claimed in claim 8 wherein one of the moiety z and the capture moiety is an antigen and the other is an antibody; or where one of z and the capture moiety is biotin or a biotin derivative and the other is avidin or streptavidin or a derivative thereof.

10. The method as claimed in any one of claims 1 to 6 wherein z comprises fluorescein, 2-oxo-2H-1-benzopyranyl or 4-methyl-2-oxo-2H-chromen-7-yl.

11. The method as claimed in any one of claims 1 to 10 wherein, in the compound of general formula (I), R¹, R², R³, R⁴ and R⁵ each independently represent hydrogen, halogen, NR⁶R⁷, C(O)NR⁶R⁷ or C₁-C₆ alkyl, -O(C₁-C₆ alkyl) or C(O)O(C₁-C₆ alkyl),any of which may optionally be substituted with one or more substituents selected from halo, hydroxy, thiol, amino, carbonyl, carboxyl, cyano, azido, C₂-C₆ alkenyl and C₂-C₆ alkynyl.

12. The method as claimed in any one of claims 1 to 11 wherein in the compound of general formula (I), independently or in any combination:
R¹, R², R³, R⁴ and R⁵ are each independently hydrogen, methyl or ethyl;
X is O or NH; and
YisO.

13. The method as claimed in claim 1 wherein the compound of formula (I) is selected from
2-Oxo-2H-1-benzopyran-7-yl 3-methyl-3-(2,4,5-trimethyl-3,6-dioxo-cyclohexa-1,4-dienyl)-butanoate (R¹= R²=R³=R⁴=R⁵=Me);
2-Oxo-2H-1-benzopyran-7-yl 3-(4,5-dimethyl-3,6-dioxo-cyclohexa-1,4-dienyl)-3-methyl-butanoate (R¹= R²=R⁴=R⁵=Me; R³=H);
2-Oxo-2H-1-benzopyran-7-yl 3-(2,4,5-trimethyl-3,6-dioxo-cyclohexa-1,4-dienyl)-propanoate (R¹=R²=R³=Me; R⁴=R⁵=H); and
4-Methyl-2-oxo-2H-chromen-7-yl 3-methyl-3-(2,4,5-trimethyl-3,6-dioxocyclohexa-1,4-dienyl)butanoate (R¹=R²=R³=R⁴=R⁵=Me).

14. The method as claimed in claim 1 wherein the patient has or is suspected of having superficial bladder tumours.

15. The method as claimed in any one of claims 1 to 14, comprising contacting the biological sample with a compound as defined in any one of claims 3 to 6 and wherein, in step (iii) determining the presence or absence of a compound or ion of the formula:
z-XH or z-X⁻
comprises detecting the presence of a chromophore or a luminophore.

16. The method as claimed in any one of claims 1 to 14, comprising contacting the biological sample with a compound as defined in claim 7 and wherein, in step (iii) determining the presence or absence of a compound of the formula:
z-XH or z-X⁻
comprises detecting the presence of a coloured or magnetic particle.

17. The method as claimed in any one of claims 1 to 14, comprising contacting the biological sample with a compound as defined in claim 8 or claim 9 and wherein, in step (iii) determining the presence or absence of a compound of the formula:
z-XH or z-X⁻
comprises detecting the presence of the compound z-XH or z-X⁻ bound to the capture moiety.

18. A method as claimed in any one of claims 14 to 17 wherein, step (iii) comprises quantitatively determining the presence or determining the absence of the compound of the formula z-XH or z-X⁻.

19. The method as claimed in claim 18 further comprising the step of determining the number of cells in the urine sample, or processed derivative thereof, whereby NQO1/NQO2 activity can be expressed per cell.

20. The method as claimed in claim 18 wherein step iii) comprises quantitatively determining the presence or absence of the compound of the formula z-XH or z-X- as a ratio of the z-XH or z-X- concentration of the urine sample to that of a negative assay control sample.

21. The method according to claim 19 wherein said ratio is correlated with known cancer cell staging techniques.

## Patentansprüche

1. Verfahren zum Bestimmen des Vorhandenseins oder Nichtvorhandenseins, in einer Urinprobe von einem Patienten, von Blasen- oder Prostatakrebszellen, die NQO1 und/oder NQO2 überexprimieren, wobei das Verfahren Folgendes umfasst:
i. Kontaktieren der Urinprobe oder eines verarbeiteten Derivats davon mit einer Verbindung der allgemeinen Formel (I), (Ia), (Ib), (Ic), (Id), (Ie) oder (If) oder gegebenenfalls einem Salz einer beliebigen derselben, wobei die Urinprobe Blasen- oder Prostatakrebszellen enthält oder im Verdacht steht, diese zu enthalten, die NQO1 und/oder NQO2 überexprimieren;
ii. gegebenenfalls, falls die Blasen- oder Prostatakrebszellen NQO2 überexprimieren oder im Verdacht stehen, NQO2 zu überexprimieren, Beigeben eines NQO2-Co-Substrats zu der Probe; und
iii. Bestimmen des Vorhandenseins oder Nichtvorhandenseins einer Verbindung der Formel:
z-XH;
oder eines Ions der Formel:
z-X⁻;
wobei z und X wie in der allgemeinen Formel (I) definiert sind, wobei das Vorhandensein der Verbindung oder des Ions auf das Vorhandensein in der Probe von Krebszellen, die NQO1 und/oder NQO2 überexprimieren, hinweist,
wobei die allgemeine Formel (I) die Folgende ist: 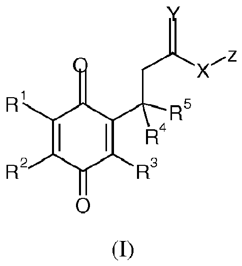
wobei R¹, R², R³, R⁴ und R⁵ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert; oder
R¹ und R² zusammen mit den Kohlenstoffatomen, an die sie gebunden sind, ein 5- oder 6-gliedriges, gegebenenfalls substituiertes aromatisches, heteroaromatisches, carbocyclisches oder heterocyclisches Ringsystem ausbilden;
X O, S oder NR⁸ ist;
R⁸ Wasserstoff oder C₁-C₃-Alkyl ist;
Y O, S oder NR⁹ ist;
R⁹ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (Ia) die Folgende ist: 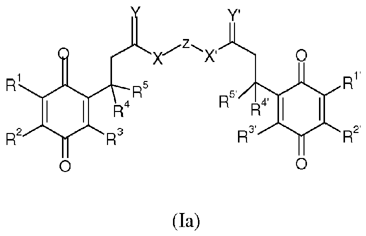
wobei R¹, R², R³, R⁴ und R⁵ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert; oder
R¹ und R² zusammen mit den Kohlenstoffatomen, an die sie gebunden sind, ein 5- oder 6-gliedriges, gegebenenfalls substituiertes aromatisches, heteroaromatisches, carbocyclisches oder heterocyclisches Ringsystem ausbilden;
R^{1'}, R^{2'}, R^{3'}, R^{4'} und R^{5'} jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert; oder
R¹¹ und R^{2'} zusammen mit den Kohlenstoffatomen, an die sie gebunden sind, ein 5- oder 6-gliedriges, gegebenenfalls substituiertes aromatisches, heteroaromatisches, carbocyclisches oder heterocyclisches Ringsystem ausbilden;
X O, S oder NR⁸ ist;
R⁸ Wasserstoff oder C₁-C₃-Alkyl ist;
Y O, S oder NR⁹ ist;
R⁹ Wasserstoff oder C₁-C₃-Alkyl ist;
X' O, S oder NR⁸ ist;
R⁸ Wasserstoff oder C₁-C₃-Alkyl ist;
Y' O, S oder NR⁹ ist;
R⁹ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (Ib) die Folgende ist: 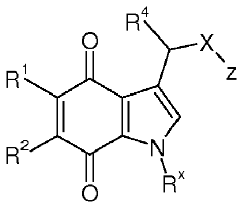
wobei R¹, R² und R⁴ unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert; oder
R¹ und R² zusammen mit den Kohlenstoffatomen, an die sie gebunden sind, ein 5- oder 6-gliedriges, gegebenenfalls substituiertes aromatisches, heteroaromatisches, carbocyclisches oder heterocyclisches Ringsystem ausbilden;
R^{x} H oder C₁-C₃-Alkyl ist;
X O, S oder NR⁸ ist;
R⁸ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (Ic) die Folgende ist: 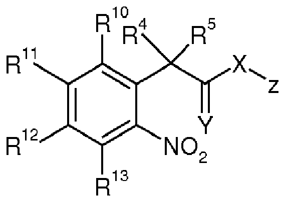
wobei R⁴, R⁵, R¹⁰, R¹¹, R¹² und R¹³ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert;
X O, S oder NR⁸ ist;
R⁸ Wasserstoff oder C₁-C₃-Alkyl ist;
Y O, S oder NR⁹ ist;
R⁹ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (Id) die Folgende ist: 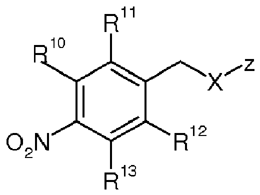
wobei X 0, S oder NR⁸ ist;
R⁸ Wasserstoff oder C₁-C₃-Alkyl ist;
R¹⁰, R¹¹, R¹² und R¹³ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (Ie) die Folgende ist: 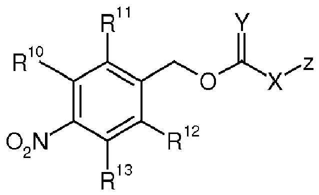
wobei R¹⁰, R¹¹, R¹² und R¹³ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert;
X O, S oder NR⁸ ist;
R⁸ Wasserstoff oder C₁-C₃-Alkyl ist;
Y O, S oder NR⁹ ist;
R⁹ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden;
die allgemeine Formel (If) die Folgende ist: 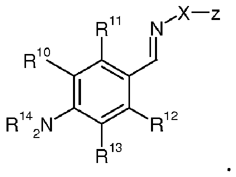
wobei R¹⁰, R¹¹, R¹² und R¹³ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren reaktiven Substituenten substituiert sein kann;
R⁶ und R⁷ jeweils unabhängig voneinander für Wasserstoff oder C₁-C₆-Alkyl stehen, gegebenenfalls mit Halogen substituiert;
R¹⁴ Wasserstoff oder C₁-C₆-Alkyl ist und
X O, S oder NR⁸ ist;
R⁸ Wasserstoff oder C₁-C₃-Alkyl ist;
z eine Gruppe ist, die kovalent mit dem Rest des Moleküls verknüpft ist und die, bei Reduktion der Verbindung der allgemeinen Formel (I), von dem Rest des Moleküls abgespalten wird, um eine nachweisbare Verbindung z-XH oder ein nachweisbares Ion z-X⁻ auszubilden.

2. Verfahren nach Anspruch 1, wobei, falls die Blasen- oder Prostatakrebszellen NQO1 überexprimieren oder im Verdacht stehen, NQO1 zu überexprimieren, ein NQO1-Co-Substrat zu der Probe beigegeben wird.

3. Verfahren nach Anspruch 1 oder 2, wobei z ein Chromophor oder Luminophor umfasst.

4. Verfahren nach Anspruch 3, wobei z einen fluoreszierenden, phosphoreszierenden, chemilumineszierenden oder biolumineszierenden Marker umfasst, sodass z-XH oder z-X⁻ ein fluoreszierendes, phosphoreszierendes, chemilumineszierendes oder biolumineszierendes Molekül oder Ion; oder ein Modulator von Emissionen von einem fluoreszierenden, phosphoreszierenden, chemilumineszierenden oder biolumineszierenden Molekül oder Ion; oder ein Co-Faktor für eine chemilumineszierende oder biolumineszierende Reaktion ist.

5. Verfahren nach Anspruch 4, wobei sich die optischen Eigenschaften der Gruppe z ändern, wenn sie von dem Rest der Verbindung der allgemeinen Formel (I) abgespalten wird, um die Verbindung z-XH oder das Ion z-X⁻ auszubilden.

6. Verfahren nach Anspruch 5, wobei die Änderung in den optischen Eigenschaften der Gruppe z eine nachweisbare Änderung in der Wellenlänge des emittierten Lichts, die Beseitigung einer Quenchwirkung, die von der Chinongruppe der allgemeinen Formel (I) ausgeübt wird, oder, im Falle von Co-Faktoren, eine Abänderung deren Aktivität umfasst.

7. Verfahren nach Anspruch 1, wobei die Gruppe z ein nachweisbares Partikel, zum Beispiel ein gefärbtes Latexmikropartikel, Goldnanopartikel oder magnetisches Partikel, umfasst.

8. Verfahren nach Anspruch 1 oder Anspruch 7, wobei die Gruppe z einen bindenden Abschnitt umfasst, der selektiv eine Fängergruppe bindet, wobei die Fängergruppe einen Bindungspartner für die Gruppe z umfasst.

9. Verfahren nach Anspruch 8, wobei die Gruppe z oder die Fängergruppe ein Antigen und die jeweils andere ein Antikörper ist; oder wobei z oder die Fängergruppe Biotin oder ein Biotinderivat und die jeweils andere Avidin oder Streptavidin oder ein Derivat desselben ist.

10. Verfahren nach einem der Ansprüche 1 bis 6, wobei z Fluoreszein, 2-Oxo-2H-1-benzopyranyl oder 4-Methyl-2-oxo-2H-chromen-7-yl umfasst.

11. Verfahren nach einem der Ansprüche 1 bis 10, wobei in der Verbindung der allgemeinen Formel (I), R¹, R², R³, R⁴ und R⁵ jeweils unabhängig voneinander für Wasserstoff, Halogen, NR⁶R⁷, C(O)NR⁶R⁷ oder C₁-C₆-Alkyl, -O(C₁-C₆-Alkyl) oder C(O)O(C₁-C₆-Alkyl) stehen, von denen jedes gegebenenfalls mit einem oder mehreren Substituenten substituiert sein kann, ausgewählt aus Halogen, Hydroxy, Thiol, Amino, Carbonyl, Carboxy, Cyano, Azido, C₂-C₆-Alkenyl und C₂-C₆-Alkinyl.

12. Verfahren nach einem der Ansprüche 1 bis 11, wobei in der Verbindung der allgemeinen Formel (I), voneinander unabhängig oder in jeder beliebigen Kombination:
R¹, R², R³, R⁴ und R⁵ jeweils voneinander unabhängig Wasserstoff, Methyl oder Ethyl stehen;
X für 0 oder NH sind; und
Y 0 ist.

13. Verfahren nach Anspruch 1, wobei die Verbindung der Formel (I) ausgewählt ist aus:
2-Oxo-2H-1-benzopyran-7-yl-3-methyl-3-(2,4,5-trimethyl-3,6-dioxocyclohexa-1,4-dienyl)butanoat (R¹=R²=R³=R⁴=R⁵=Me) ;
2-Oxo-2H-1-benzopyran-7-yl-3-(4,5-dimethyl-3,6-dioxocyclohexa-1,4-dienyl)-3-methylbutanoat (R¹=R²=R⁴=R⁵=Me; R³=H);
2-Oxo-2H-1-benzopyran-7-yl-3-(2,4,5-trimethyl-3,6-dioxocyclohexa-1,4-dienyl)propanoat (R¹=R²=R³=Me; R⁴=R⁵=H); und
4-Methyl-2-oxo-2H-chromen-7-yl-3-methyl-3-(2,4,5-trimethyl-3,6-dioxocyclohexa-1,4-dienyl)butanoat (R¹=R²=R³=R⁴=R⁵=Me).

14. Verfahren nach Anspruch 1, wobei der Patient oberflächliche Blasentumoren hat oder im Verdacht steht, diese zu haben.

15. Verfahren nach einem der Ansprüche 1 bis 14, umfassend Kontaktieren der biologischen Probe mit einer wie in einem der Ansprüche 3 bis 6 definierten Verbindung und wobei, in Schritt (iii), Bestimmen des Vorhandenseins oder Nichtvorhandenseins einer Verbindung oder eines Ions der Formel:
z-XH
oder
z-X⁻
Nachweisen des Vorhandenseins eines Chromophors oder eines Luminophors umfasst.

16. Verfahren nach einem der Ansprüche 1 bis 14, umfassend Kontaktieren der biologischen Probe mit einer wie in Anspruch 7 definierten Verbindung und wobei, in Schritt (iii), Bestimmen des Vorhandenseins oder Nichtvorhandenseins einer Verbindung der Formel:
z-XH
oder
z-X⁻
Nachweisen des Vorhandenseins eines gefärbten oder magnetischen Partikels umfasst.

17. Verfahren nach einem der Ansprüche 1 bis 14, umfassend Kontaktieren der biologischen Probe mit einer wie in Anspruch 8 oder Anspruch 9 definierten Verbindung und wobei, in Schritt (iii), Bestimmendes Vorhandenseins oder Nichtvorhandenseins einer Verbindung der Formel:
z-XH
oder
z-X⁻
Nachweisen des Vorhandenseins der Verbindung z-XH oder z-X⁻, gebunden an die Fängergruppe, umfasst.

18. Verfahren nach einem der Ansprüche 14 bis 17, wobei Schritt (iii) quantitatives Bestimmen des Vorhandenseins oder Bestimmen des Nichtvorhandenseins der Verbindung der Formel z-XH oder z-X⁻ umfasst.

19. Verfahren nach Anspruch 18, ferner den Schritt des Bestimmens der Anzahl an Zellen in der Urinprobe oder eines verarbeiteten Derivats derselben umfassend, wobei die NQO1/NQO2-Aktivität pro Zelle exprimiert sein kann.

20. Verfahren nach Anspruch 18, wobei Schritt iii) quantitatives Bestimmen des Vorhandenseins oder Nichtvorhandenseins der Verbindung der Formal z-XH oder z-X⁻ als ein Verhältnis der z-XH- oder z-X⁻-Konzentration der Urinprobe zu jener einer negativen Assaykontrollprobe umfasst.

21. Verfahren nach Anspruch 19, wobei das Verhältnis mit bekannten Stadieneinteilungstechniken für Krebszellen korreliert.

## Revendications

1. Méthode de détermination de la présence ou de l'absence, dans un échantillon d'urine provenant d'un patient, de cellules de cancer de la vessie ou de la prostate qui surexpriment NQO1 et/ou NQO2, la méthode comprenant :
i. la mise en contact de l'échantillon d'urine, ou de l'un de ses dérivés traités, avec un composé de formule générale (I), (Ia), (Ib), (Ic), (Id), (Ie) ou (If) ou un sel de l'un quelconque d'entre eux lorsque c'est applicable, où l'échantillon d'urine contient ou est suspecté de contenir des cellules de cancer de la vessie ou de la prostate qui surexpriment NQO1 et/ou NQO2 ;
ii. éventuellement, dans le cas où les cellules de cancer de la vessie ou de la prostate surexpriment ou sont suspectées de surexprimer NQO2, l'addition d'un co-substrat de NQO2 à l'échantillon ; et
iii. la détermination de la présence ou de l'absence d'un composé de formule :
z-XH ;
ou d'un ion de formule :
z-X⁻ ;
où z et X sont tels que définis dans la formule générale (I), où la présence du composé ou de l'ion indique la présence dans l'échantillon de cellules cancéreuses qui surexpriment NQO1 et/ou NQO2
où la formule générale (I) est : 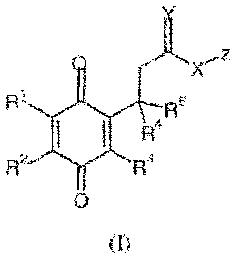
dans laquelle R¹, R², R³, R⁴ et R⁵ chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ; ou
R¹ et R² conjointement avec les atomes de carbone auxquels ils sont fixés forment un système de cycle aromatique, hétéroaromatique, carbocyclique ou hétérocyclique de 5 ou 6 chaînons éventuellement substitué ;
X représente O, S ou NR⁸ ;
R⁸ représente un atome d'hydrogène ou un groupe C₁-C₃-alkyle ;
Y représente O, S ou NR₉ ;
R⁹ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (Ia) est : 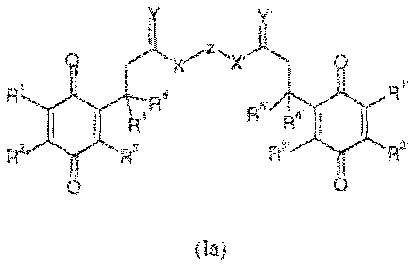
dans laquelle R¹, R², R³, R⁴ et R⁵ chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ; ou
R¹ et R² conjointement avec les atomes de carbone auxquels ils sont fixés forment un système de cycle aromatique, hétéroaromatique, carbocyclique ou hétérocyclique de 5 ou 6 chaînons éventuellement substitué ;
R^{1'}, R^{2'}, R^{3'}, R^{4'} et R^{5'} chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ; ou
R^{1'} et R^{2'} conjointement avec les atomes de carbone auxquels ils sont fixés forment un système de cycle aromatique, hétéroaromatique, carbocyclique ou hétérocyclique de 5 ou 6 chaînons éventuellement substitué ;
X représente O, S ou NR⁸ ;
R⁸ représente un atome d'hydrogène ou un groupe C₁-C₃-alkyle ;
Y représente O, S ou NR⁹ ;
R⁹ représente un atome d'hydrogène ou un groupe C₁-C₃-alkyle ;
X' représente O, S ou NR⁸ ;
R⁸ représente un atome d'hydrogène ou un groupe C₁-C₃-alkyle ;
Y' représente O, S ou NR⁹ ;
R⁹ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (Ib) est : 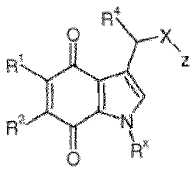
dans laquelle R¹, R², et R⁴
chacun indépendamment représentent un atome d'hydrogène, d'halogène, un groupe NR⁶R⁷, C(O)NR⁶R⁷ ou C₁-C₆-alkyle, - O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ; ou
R¹ et R² conjointement avec les atomes de carbone auxquels ils sont fixés forment un système de cycle aromatique, hétéroaromatique, carbocyclique ou hétérocyclique de 5 ou 6 chaînons éventuellement substitué ;
R^{x} représente H, ou un groupe C₁-C₃-alkyle ;
X représente O, S ou NR⁸ ;
R⁸ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (Ic) est : 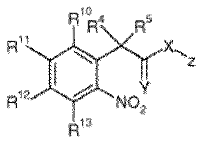
dans laquelle R⁴, R⁵, R¹⁰, R¹¹, R¹² et R¹³ chacun indépendamment représentent un atome d'hydrogène, d'halogène, un groupe NR⁶R⁷, C(O)NR⁶R⁷ ou C₁-C₆-alkyle, - O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ;
X représente O, S ou NR⁸ ;
R⁸ représente un atome d'hydrogène ou un groupe C₁-C₃-alkyle ;
Y représente O, S ou NR⁹ ;
R⁹ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (Id) est : 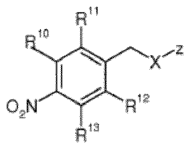 dans laquelle :
X représente O, S ou NR⁸ ;
R⁸ représente un atome d'hydrogène ou un groupe C₁-C₃-alkyle ;
R¹⁰, R¹¹, R¹² et R¹³ chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (Ie) est : 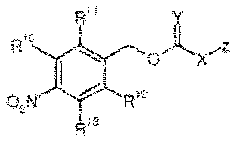 dans laquelle :
R¹⁰, R¹¹, R¹² et R¹³ chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ;
X représente O, S ou NR⁸ ;
R⁸ représente un atome d'hydrogène ou un groupe C₁-C₃-alkyle ;
Y représente O, S ou NR⁹ ;
R⁹ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable ;
la formule générale (If) est : 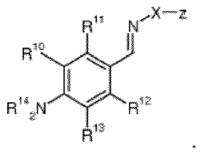 dans laquelle :
R¹⁰, R¹¹, R¹² et R¹³ chacun indépendamment représentent un atome d'hydrogène, d'halogène, un groupe NR⁶R⁷, C(O)NR⁶R⁷ ou C₁-C₆-alkyle, -O(C₁-C₆-alkyle) ou C(O)O(C₁-C₆-alkyle), dont l'un quelconque d'entre eux peut être éventuellement substitué par un ou plusieurs substituants réactifs ;
R⁶ et R⁷ chacun indépendamment représentent un atome d'hydrogène ou un groupe C₁-C₆-alkyle éventuellement substitué par un groupe halogéno ;
R¹⁴ représente H ou un groupe C₁-C₆-alkyle et
X représente O, S ou NR⁸ ;
R⁸ représente un atome d'hydrogène ou un groupe C₁-C₃-alkyle ;
z représente un radical qui est lié de façon covalente au reste de la molécule et qui, lors de la réduction du composé de formule générale (I), est clivé du reste de la molécule pour former un composé z-XH ou un ion z-X⁻ détectable.

2. Méthode telle que revendiquée dans la revendication 1 dans laquelle, dans le cas où les cellules de cancer de la vessie ou de la prostate surexpriment ou sont suspectées de surexprimer NQO1, l'addition d'un co-substrat de NQO1 à l'échantillon.

3. Méthode telle que revendiquée dans la revendication 1 ou 2, dans laquelle z comprend un chromophore ou un luminophore.

4. Méthode telle que revendiquée dans la revendication 3 dans laquelle z comprend un marqueur fluorescent, phosphorescent, chimioluminescent ou bioluminescent de telle façon que z-XH or z-X⁻ est une molécule ou un ion fluorescent, phosphorescent, chimioluminescent ou bioluminescent ; ou un modulateur des émissions provenant d'une molécule ou d'un ion fluorescent, phosphorescent, chimioluminescent ou bioluminescent ; ou un cofacteur pour une réaction chimioluminescente ou bioluminescente.

5. Méthode telle que revendiquée dans la revendication 4 dans laquelle les propriétés optiques du radical z changent lorsqu'il est clivé du reste du composé de formule générale (I) pour former le composé z-XH ou l'ion z-X⁻.

6. Méthode telle que revendiquée dans la revendication 5, dans laquelle le changement des propriétés optiques du radical z comprennent un changement détectable de la longueur d'onde de la lumière émise, l'élimination d'un effet d'extinction exercé par le radical quinone de formule générale (I) ou, dans le cas de cofacteurs, une modulation de leur activité.

7. Méthode telle que revendiquée dans la revendication 1 dans laquelle le radical z comprend une particule détectable, par exemple une microparticule de latex colorée, une nanoparticule d'or ou une particule magnétique.

8. Méthode telle que revendiquée dans la revendication 1 ou la revendication 7 dans laquelle le radical z comprend une partie de liaison qui lie sélectivement un radical de capture où le radical de capture comprend un partenaire de liaison pour le radical z.

9. Méthode telle que revendiquée dans la revendication 8 dans laquelle l'un du radical z et du radical de capture est un antigène et l'autre est un anticorps ; ou bien où l'un de z et du radical de capture est la biotine ou un dérivé de la biotine et l'autre est l'avidine ou la streptavidine ou l'un de leurs dérivés.

10. Méthode telle que revendiquée dans l'une quelconque des revendications 1 à 6 dans laquelle z comprend la fluorescéine, le 2-oxo-2H-1-benzopyranyle ou le 4-méthyl-2-oxo-2H-chromén-7-yle.

11. Méthode telle que revendiquée dans l'une quelconque des revendications 1 à 10 dans laquelle, dans le composé de formule générale (I), R¹, R², R³, R⁴ et R⁵ chacun indépendamment représentent un atome d'hydrogène, d'halogène, un groupe NR⁶R⁷, C(O)NR⁶R⁷ ou C₁-C₆-alkyle,-O(C₁-C₆-alkyle) ou C(O)O(C₁-C₆-alkyle), dont l'un quelconque d'entre eux peut être éventuellement substitué par un ou plusieurs substituants sélectionnés parmi les groupes halogéno, hydroxy, thiol, amino, carbonyle, carboxyle, cyano, azido, C₂-C₆-alcényle et C₂-C₆-alcynyle.

12. Méthode telle que revendiquée dans l'une quelconque des revendications 1 à 11 dans laquelle dans le composé de formule générale (I), indépendamment ou dans toute combinaison :
R¹, R², R³, R⁴ et R⁵ représentent chacun indépendamment un atome d'hydrogène, un groupe méthyle ou éthyle ;
X représente O ou NH ; et
Y représente O.

13. Méthode telle que revendiquée dans la revendication 1 dans laquelle le composé de formule (I) est sélectionné parmi
le 3-méthyl-3-(2,4,5-triméthyl-3,6-dioxo-cyclohexa-1,4-diényl)-butanoate de 2-oxo-2H-1-benzopyran-7-yle (R¹=R²=R³=R⁴=R⁵=Me) ;
le 3-(4,5-diméthyl-3,6-dioxo-cyclohexa-1,4-diényl)-3-méthyl-butanoate de 2-oxo-2H-1-benzopyran-7-yle (R¹=R²=R⁴=R⁵=Me ; R³=H) ;
le 3-(2,4,5-triméthyl-3,6-dioxo-cyclohexa-1,4-diényl)-propanoate de 2-oxo-2H-1-benzopyran-7-yle (R¹=R²=R³=Me ; R⁴=R⁵=H) ; et
le 3-méthyl-3-(2,4,5-triméthyl-3,6-dioxocyclohexa-1,4-diényl)butanoate de 4-méthyl-2-oxo-2H-chromén-7-yle (R¹=R²=R³=R⁴=R⁵=Me).

14. Méthode telle que revendiquée dans la revendication 1 dans laquelle le patient souffre ou est suspecté de souffrir de tumeurs superficielles de la vessie.

15. Méthode telle que revendiquée dans l'une quelconque des revendications 1 à 14, comprenant la mise en contact de l'échantillon biologique avec un composé tel que défini dans l'une quelconque des revendications 3 à 6 et dans laquelle, dans l'étape (iii) la détermination de la présence ou de l'absence d'un composé ou d'un ion de formule :
z-XH
ou
z-X⁻
comprend la détection de la présence d'un chromophore ou d'un luminophore.

16. Méthode telle que revendiquée dans l'une quelconque des revendications 1 à 14, comprenant la mise en contact de l'échantillon biologique avec un composé tel que défini dans la revendication 7 et dans laquelle, dans l'étape (iii) la détermination de la présence ou de l'absence d'un composé de formule :
z-XH
ou
z-X⁻
comprend la détection de la présence d'une particule colorée ou magnétique.

17. Méthode telle que revendiquée dans l'une quelconque des revendications 1 à 14, comprenant la mise en contact de l'échantillon biologique avec un composé tel que défini dans la revendication 8 ou la revendication 9 et dans laquelle, dans l'étape (iii) la détermination de la présence ou de l'absence d'un composé de formule :
z-XH
ou
z-X⁻
comprend la détection de la présence du composé z-XH ou z-X⁻ lié au radical de capture.

18. Méthode telle que revendiquée dans l'une quelconque des revendications 14 à 17 dans laquelle, l'étape (iii) comprend la détermination quantitative de la présence ou la détermination de l'absence du composé de formule z-XH ou z-X⁻.

19. Méthode telle que revendiquée dans la revendication 18 comprenant en outre l'étape de détermination du nombre de cellules dans l'échantillon d'urine ou l'un de ses dérivés traités, par lequel l'activité NQO1/NQO2 peut être exprimée par cellule.

20. Méthode telle que revendiquée dans la revendication 18 dans laquelle l'étape (iii) comprend la détermination quantitative de la présence ou de l'absence du composé de formule z-XH ou z-X⁻ sous la forme d'un rapport de la concentration de z-XH ou z-X- de l'échantillon d'urine sur celle d'un échantillon témoin négatif du test.

21. Méthode selon la revendication 19 dans laquelle ledit rapport est corrélé avec des techniques connues de détermination du stade de cellules cancéreuses.
